# FASCICULE DE BREVET EUROPEEN

(11) **EP 1 702 611 B1**
(45) Date de publication et mention de la délivrance du brevet: **11.11.2009**
(21) Numéro de dépôt: 06110407.1
(22) Date de dépôt: 24.02.2006
(51) Int. Cl.: A61K 8/49, A61Q 5/06, A61Q 5/10

(54) **Composition de teinture des fibres kératiniques comprenant un colorant direct sulfonamidoxanthenique et procédé de teinture la mettant en oeuvre**
Mittel zum Färben keratinischer Fasern enthaltend einen direktziehenden Sulfonamidxanthenylfarbstoff und Färbeverfahren
Composition for dyeing keratinuous fibres comprising a direct sulfonamidoxanthenyl dye and dyeing process

(30) Priorité: 25.02.2005 FR 0550522
(43) Date de publication de la demande: 20.09.2006
(73) Titulaire: L'ORÉAL, 75008 Paris (FR)
(72) Inventeur: Lagrange, Alain, 77700, Coupvray (FR)
(74) Mandataire: Poulin, Gérard

(56) Documents cités:
- EP-A- 1 199 065
- GB-A- 2 259 717
- US-A1- 2004 231 069

## Description

La présente invention concerne une composition de teinture des fibres kératiniques, en particulier des fibres kératiniques humaines et, plus particulièrement, des cheveux, qui comprend au moins un composé choisi parmi les colorants directs de type sulfonamidoxanthénique autrement dit les dérivés sulfonamides de xanthènes.

L'invention concerne, en outre, un procédé de teinture des fibres kératiniques, en particulier des fibres kératiniques humaines et, plus particulièrement, des cheveux qui met en oeuvre ladite composition.

Il existe de nombreuses compositions et de nombreux procédés pour teindre les fibres kératiniques et, en particulier, les cheveux humains.

Ainsi, il est connu de teindre les fibres kératiniques et, en particulier, les cheveux humains avec des compositions tinctoriales contenant des précurseurs de colorant d'oxydation, en particulier des ortho ou paraphénylènediamines, des ortho ou paraaminophénols, des composés hétérocycliques, tels que des dérivés de diaminopyrazole, appelés généralement « bases d'oxydation ». Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu, d'une part, au niveau des bases d'oxydation et, d'autre part, au niveau des coupleurs, permet l'obtention d'une riche palette de couleurs.

La coloration, dite « permanente », obtenue grâce à ces colorants d'oxydation, doit, par ailleurs, satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, l'ondulation permanente, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles, tout au long d'une même fibre kératinique, qui peut être en effet différemment sensibilisée, c'est-à-dire abîmée entre sa pointe et sa racine.

Il s'avère, en conclusion, que si les associations base-coupleur classiques permettent d'obtenir une palette étendue de couleurs, elles ne permettent pas de satisfaire aux critères énumérés plus haut et, notamment, conduisent souvent à la génération dans la fibre de produits de couplage variés posant des problèmes de ténacité difficiles à maîtriser, comme par exemple un virage sélectif.

Une autre manière de teindre les fibres kératiniques, en particulier les cheveux, est d'utiliser des colorants directs.

Les colorants classiques qui sont utilisés sont en particulier des colorants du type nitrés benzèniques, anthraquinoniques, nitropyridiniques, azoïques, azoïques cationiques, xanthéniques, acridiniques aziniques, triarylméthanes, benzéniques nitrés ou des colorants naturels.

Ces colorants qui sont des molécules colorées et colorantes ayant une affinité pour les fibres sont appliqués sur les fibres kératiniques pendant un temps nécessaire à l'obtention de la coloration désirée, puis rincés.

La coloration directe est de ce fait très répandue car elle présente, en outre, certains avantages par rapport aux précurseurs de coloration d'oxydation et, notamment, souvent une meilleure inocuité et l'absence de sensibilisation du cheveu due au processus oxydatif.

Les colorations obtenues sont cependant temporaires ou semi-permanentes, car la nature des interactions qui lient les colorants directs à la fibre kératinique, et leur désorption de la surface et/ou du coeur de la fibre sont responsables de leur faible puissance tinctoriale et de leur mauvaise tenue aux lavages, aux intempéries, ou à la transpiration. Ces colorants directs sont en outre généralement sensibles à la lumière du fait de la faible résistance du chromophore vis-à-vis des attaques photochimiques et conduisent dans le temps à un affadissement de la coloration des cheveux.

Il existe donc un besoin pour une composition de teinture des fibres kératiniques, en particulier des fibres kératiniques humaines et, plus particulièrement, des cheveux, qui soit d'une innocuité, d'une compatibilité, totale, vis-à-vis des fibres kératiniques, qui soit peu sélective, qui donne une grande variété de couleurs, puissantes, et qui permette, en outre, d'obtenir une coloration des fibres kératiniques tenace, stable, résistante aux agents extérieurs, tels que la lumière, les intempéries, le lavage, la transpiration, et les frottements et aux traitements ultérieurs, tels que l'ondulation permanente.

Il existe encore un besoin pour une composition de teinture qui permette le traitement de toutes sortes de fibres kératiniques, de tous types de cheveux, par exemple des cheveux blancs, même si ces cheveux ont subi préalablement un traitement, tel qu'un traitement de décoloration ou d'ondulation permanente.

Il existe enfin un besoin pour une composition qui réalise la teinture sans qu'il soit nécessaire de réaliser, au préalable, une sensibilisation de la fibre kératinique, du cheveu.

Le but de la présente invention est de fournir une composition de teinture des fibres kératiniques, en particulier des fibres kératiniques humaines et, plus particulièrement, des cheveux, qui réponde, entre autres, aux besoins énumérés ci-dessus.

Le but de la présente invention est encore de fournir une composition de teinture des fibres kératiniques qui ne présente pas les inconvénients, défauts, limitations et désavantages des compositions de teinture de l'art antérieur, qu'il s'agisse des compositions de teinture mettant en oeuvre une association base-coupleur, ou des compositions mettant en oeuvre un colorant direct.

Ce but et d'autres encore sont atteints, conformément à l'invention, par une composition pour la teinture des fibres kératiniques comprenant dans un milieu cosmétique approprié pour la teinture, au moins un colorant choisi parmi les composés répondant à la formule (I) suivante, leurs formes mésomères et leurs oligomères : 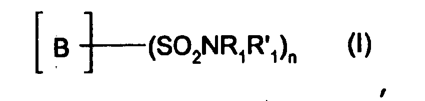 dans laquelle B représente : 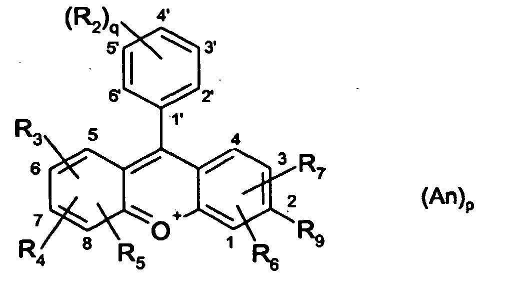 dans laquelle R₁, R'₁, identiques ou non, représentent :
o un atome d'hydrogène,
o un radical alkyle,
o un radical thioalkyle,
o un radical alcoxy,
o un radical amino alkyle,
o un radical trialkylammonioalkyle,
o un radical carboxy alkyle,
o un radical carboxy amino alkyle,
o un radical amino alcoxy polyalcoxy *(motif oxyde d'éthylène ou de propylène : (H₂N-RO(R'O)n-)* dans lequel les radicaux alcoxy du groupement polyalcoxy, identiques ou non, comprennent chacun 1 à 4 atomes de carbone,
o un radical alcoxycarbonyle,
o un radical alcoxycarbonylalkyle,
o un radical alcoxyalkyle,
o un radical acyle,
o un radical aryle, de préférence phényle,
o un radical arylalkyle, de préférence benzyle,
o un hétérocycle avec un atome d'azote, à 5 à 12 chaînons, de préférence 5 à 7 chaînons, saturé ou non, comprenant éventuellement au moins un autre hétéroatome choisi parmi l'oxygène, l'azote ou le soufre, ledit hétérocycle étant éventuellement substitué ;
   R'₁ pouvant également représenter le groupement suivant : 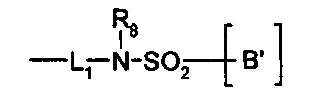
dans laquelle :
- R₈, représente un groupement choisi parmi ceux définissant R₁ et R'₁,
- L₁ représente une chaîne hydrocarbonée divalente comprenant de 1 à 16 atomes de carbone, éventuellement interrompue par au moins un hétéroatome choisi parmi l'oxygène, le soufre ou l'azote, et/ou par un groupement comprenant au moins un hétéroatome, tel que par exemple CO, SO₂,
- B' représente un groupement de formule (B) et est identique ou non à B ;
les radicaux R₁ et R'₁ forment éventuellement ensemble avec l'atome d'azote auquel ils sont rattachés, un hétérocycle, de préférence saturé, comprenant de 5 à 12 chaînons, de préférence de 5 à 7 chaînons, comprenant éventuellement un autre hétéroatome, choisi parmi l'oxygène, l'azote ou le soufre, ledit hétérocycle étant éventuellement substitué;
- R₂ représente un groupement choisi parmi l'atome d'hydrogène, les atomes d'halogène tels que F, Cl, Br et I, les radicaux alcoxy, les radicaux alkyle, le radical sulfonique, le radical carboxy ;
- R₃, R₄, R₅, R₆, R₇, R₉ représentent chacun indépendamment un groupement choisi parmi l'atome d'hydrogène ; les atomes d'halogène tels que F, Cl, Br et I ; les radicaux alcoxy ; les radicaux alkyle ; les groupes amino ; les groupes amino, mono ou disubstitués par un groupe choisi parmi le groupe benzyle, les groupes aryle, et les groupes alkyle, tels que les groupes monoaryl amino, diaryl amino, monoalkylamino, dialkylamino dans lesquels les substituants du groupe amino tels que les radicaux alkyle, peuvent former ensemble avec l'atome d'azote auquel ils sont liés un hétérocycle, de préférence saturé de 5 à 12 chaînons, de préférence de 5 à 7 chaînons comprenant éventuellement au moins un autre hétéroatome choisi parmi l'azote, l'oxygène et le soufre, ledit hétérocycle pouvant être éventuellement substitué ; ou bien un ou les deux substituants du groupe amino tels que les radicaux alkyle peuvent former ensemble avec un substituant situé en position ortho du groupe amino mono ou disubstitué tel que monoalkylamino ou (dialkyl)amino un hétérocycle à 5 à 6 chaînons pouvant éventuellement comprendre en outre au moins un autre hétéroatome choisi parmi les atomes d'azote, d'oxygène et de soufre, ledit hétérocycle pouvant être éventuellement substitué ;
   le ou les groupements -SO₂NR₁R'₁ et/ou -L₁NR₈SO₂- étant rattachés soit directement sur un ou plusieurs des cycles porteurs des substituants R₂ à R₉ ou par l'intermédiaire d'un ou plusieurs des substituants R₂ à R₉ ;
   le coefficient n est un entier allant de 1 à 4, de préférence égal à 1 ;
   le coefficient q est un entier allant de 1 à 3, de préférence égal à 1 ;
   An représente un anion mono ou polyvalent, ou un mélange d'anions ; le coefficient p est égal à 0 ou 1 ; An et p étant tels que le composé soit électriquement neutre.
   Dans la formule (I) ci-dessus, le terme alkyle utilisé pour les radicaux alkyle, ainsi que pour les groupements comportant une partie alkyle, signifie, sauf indication différente, une chaîne carbonée, linéaire ou ramifiée, ou cyclique, saturée, comportant de 1 à 30 atomes de carbone, de préférence de 1 à 24 atomes de carbone, de préférence encore de 1 à 16, mieux de 1 à 8, mieux encore de 1 à 4, pouvant être éventuellement substituée par un ou plusieurs groupes identiques ou différents, choisis parmi les atomes d'halogène, tel le chlore, le brome, l'iode et le fluor ; les hétérocycles ; les radicaux aryle ; hydroxyle ; alcoxy ; amino ; acyle en C₁-C₈ ; carboxamido ; -CO₂H ; -SO₃H ; -PO₃H₂ ; -PO₄H₂ ; -NHSO₃H ; sulfonamide ; monoalkylamino ; trialkylammonium ; ou bien encore par un radical dialkylamino, dans lequel les deux groupements alkyle peuvent former, conjointement avec l'atome d'azote dudit groupement dialkyl amino, auquel ils sont liés, un hétérocycle comprenant de 5 à 7 chaînons pouvant éventuellement comprendre en outre au moins un autre hétéroatome choisi parmi les atomes d'azote, d'oxygène ou de soufre, ledit hétérocycle pouvant être éventuellement substitué.

Dans le cas où le radical alkyle est un radical cyclique, il a généralement de 3 à 30 atomes de carbone, de préférence de 4 à 16 atomes de carbone, mieux de 4 à 8 atomes de carbone, mieux encore de 4 à 7 atomes de carbone et ne comprend pas de double liaison carbone-carbone.

- De même, selon l'invention, le terme alcoxy utilisé pour les radicaux alcoxy ainsi que pour les groupements comportant une partie alcoxy, signifie, sauf indication différente, une chaîne O-alkyle, le terme alkyle ayant la signification indiquée ci-dessus.

Selon l'invention, on entend par hétérocycle, un cycle, saturé ou non, aromatique ou non contenant 5 à 12 chaînons, de préférence de 5 à 7 chaînons, et de préférence de 1 à 3 hétéroatomes choisis parmi les atomes d'azote, de soufre et d'oxygène. Ces hétérocycles peuvent être condensés sur d'autres hétérocycles, ou sur d'autres cycles notamment aromatiques tels qu'un groupement phényle. Ces hétérocycles peuvent, en outre, être quaternisés notamment par un radical alkyle.

Parmi, les hétérocycles, condensés ou non, on peut notamment citer à titre d'exemple les cycles : thiophène, benzothiophène, furane, benzofurane, indole, indoline, carbazole, pyridine, déhydroquinoléine, chromone, julodinine, thiadiazole, triazole, isoxazole, oxazole, thiazole, isothiazole, imidazole, pyrazole, triazine, thiazine, pyrazine, pyridazine, pyrimidine, pyridine, diazépine, oxazépine, benzotriazole, benzoxazole, benzimidazole, benzothiazole, morpholine, pipéridine, pipérazine, azétidine, pyrrolidine, aziridine.

Selon l'invention, il est précisé que l'hétérocycle peut éventuellement être substitué. Dans ce cas, il porte un ou plusieurs substituants, identiques ou non, choisis parmi les radicaux alkyle linéaires ou ramifiés en C₁-C₁₆, de préférence en C₁-C₁₀, hydroxyalkyle linéaires ou ramifiés en C₁-C₁₆, de préférence en C₁-C₁₀, carboxy, alcoxycarbonyle linéaires ou ramifiés dont l'alcoxy est en C₁-C₁₆, de préférence en C₁-C₁₀, aminoalkyle carbamoyle (H₂N-alkyle-NH-CO-), dans lequel la partie alkyle est linéaire ou ramifiée en C₁-C₁₆, de préférence en C₁-C₁₀.

Selon l'invention, on entend par aryle, sauf spécifié autrement, un radical aryle en C₆ à C₃₀ pouvant être substitué par un ou plusieurs groupements identiques ou différents, choisis parmi les atomes d'halogène ; les radicaux alkyle linéaire ou ramifié en C₁ à C₁₆, de préférence en C₁ à C₁₀ ; les radicaux alcoxy linéaire ou ramifié en C₁ à C₁₆, de préférence en C₁-C₁₀ ; les radicaux aryloxy éventuellement substitués ; mésyle (CH₃-SO₂-) ; cyano ; carboxamido ; -CO₂H ; sulfo (SO₃H) ; -PO₃H₂ ; -PO₄H₂ ; hydroxyle ; amino ; amino mono ou disubstitué tel que monoalkyl (C₁-C₄) amino ou dialkyl(C₁-C₄) amino, dans lequel les substituants du groupe amino tel que les groupements alkyle peuvent former, conjointement avec l'atome d'azote dudit groupement amino mono ou disubstitué tel que dialkyl(C₁-C₄)amino, auquel ils sont rattachés, un hétérocycle de préférence saturé, comprenant de 5 à 12 chaînons, de préférence de 5 à 7 chaînons, comprenant éventuellement un autre hétéroatome, choisi parmi l'oxygène, l'azote et le soufre ; ledit hétérocycle peut éventuellement être substitué, ledit hétérocycle peut éventuellement être condensé avec un cycle aromatique de préférence à 6 chaînons, le cycle aromatique étant éventuellement substitué comme précédemment indiqué.

De préférence, le groupe aryle est un groupe phényle ou un groupe naphtyle pouvant être substitué comme indiqué ci-dessus.

De préférence, dans la formule (I) ci-dessus, R₁, R'₁, identiques ou non, représentent :
o un atome d'hydrogène,
o un radical alkyle en C₁-C₁₆, linéaire ou ramifié, ou cyclique en C₃-C₁₆, éventuellement substitué par un ou plusieurs groupements identiques ou non, choisis parmi les groupements amino, sulfonique, et alcoxy,
o un radical hydroxyalkyle en C₁-C₁₆, linéaire, ramifié ou cyclique en C₃-C₁₆,
o un radical thioalkyle en C₁-C₁₆, linéaire, ramifié ou cyclique en C₃-C₁₆,
o un radical alcoxy en C₁-C₁₆, linéaire ou ramifié,
o un radical amino alkyle en C₁-C₁₆, linéaire ou ramifié, ou cyclique en C₃-C₁₆,
o un radical trialkylammonioalkyle dans lequel les radicaux alkyle, identiques ou non, sont en C₁-C₁₆,
o un radical halogénoalkyle linéaire, ou ramifié dans lequel le radical alkyle linéaire, ramifié comprend 1 à 16 atomes de carbone,
o un radical carboxy alkyle en C₁-C₁₆, linéaire ou ramifié, ou cyclique en C₃-C₁₆,
o un radical carboxy amino alkyle en C₁-C₁₆, linéaire ou ramifié, ou cyclique en C₃-C₁₆,
o un radical amino alcoxy polyalcoxy dans lequel les radicaux alcoxy, identiques ou non, linéaires ou ramifiés comprennent 1 à 4 atomes de carbone,
o un radical alcoxycarbonyle dans lequel le radical alcoxy linéaire, ou ramifié comprend 1 à 16 atomes de carbone,
o un radical alcoxycarbonylalkyle dans lequel le radical alcoxy linéaire, ou ramifié comprend 1 à 16 atomes de carbone et le radical alkyle linéaire ramifié comprend 1 à 16 atomes de carbone,
o un radical alcoxyalkyle dans lequel le radical alcoxy linéaire, ou ramifié comprend 1 à 16 atomes de carbone, et le radical alkyle linéaire ou ramifié comprend 1 à 16 atomes de carbone,
o un radical acyle dans lequel le radical alkyle linéaire, ou ramifié comprend 1 à 16 atomes de carbone,
o un radical aryle, de préférence phényle, éventuellement substitué par un ou plusieurs radicaux choisis parmi les radicaux amino, sulfo, alcoxy,
o un radical arylalkyle, de préférence benzyle, éventuellement substitué par un ou plusieurs radicaux choisis parmi les radicaux amino, sulfo, alcoxy,
o un hétérocycle avec un atome d'azote, à 5 à 12 chaînons, de préférence 5 à 7 chaînons, saturé ou non, comprenant éventuellement au moins un autre hétéroatome choisi parmi l'oxygène, l'azote ou le soufre, ledit hétérocycle étant éventuellement substitué par un ou plusieurs groupement(s) identique(s) ou non choisis parmi les radicaux alkyle ou hydroxyalkyle en C₁-C₁₆, linéaires ou ramifiés, les groupements carboxy, les groupements alcoxy carbonyle linéaires ou ramifiés dont l'alcoxy est en C₁-C₁₆, les groupements aminoalkylcarbamoyle avec le radical alkyle en C₁-C₁₆, linéaire ou ramifié.

De préférence, R₂ représente un groupement choisi parmi l'atome d'hydrogène, les atomes d'halogènes tels que F, Cl, Br et I, les radicaux alcoxy, les radicaux alkyle, le radical sulfonique, le radical carboxy.

De préférence, R₃, R₄, R₅, R₆, R₇, R₉ identiques ou non représentent chacun indépendamment un groupement choisi parmi :
o l'atome d'hydrogène ;
o les atomes d'halogène tels que F, Cl, Br et I ;
o les radicaux alcoxy ;
o les radicaux alkyle ;
o les groupes amino ; les groupes amino, mono ou disubstitués par un ou plusieurs groupe(s) identiques ou non, choisi(s) parmi le groupe benzyle, les groupes aryle et les groupes alkyle, tels que les groupes monoaryl amino, diaryl amino, monoalkylamino, (dialkyl)amino dans lesquels les substituants du groupe amino tels que les radicaux alkyle, peuvent former ensemble avec l'atome d'azote auquel ils sont rattachés un hétérocycle de préférence saturé de 5 à 12 chaînons, de préférence de 5 à 7 chaînons comprenant éventuellement en outre au moins un autre hétéroatome choisi parmi les atomes d'azote, d'oxygène et de soufre, ledit hétérocycle pouvant être éventuellement substitué ; ou bien un ou les deux substituants du groupe amino tels que les radicaux alkyle peuvent former ensemble avec un substituant situé en position ortho du groupe amino mono ou disubstitué tel que monoalkylamino ou (dialkyl)amino un hétérocycle comprenant de 5 à 7 chaînons pouvant éventuellement comprendre en outre un ou plusieurs autres hétéroatomes choisis parmi les atomes d'azote, d'oxygène et de soufre, ledit hétérocycle pouvant être éventuellement substitué ;
o les radicaux sulfoalkyle linéaire ou ramifié en C₁-C₁₆, de préférence en C₁-C₁₀ ;
o les radicaux hydroxyalkyle linéaire ou ramifiée en C₁-C₁₆, de préférence en C₁-C₁₀ ;
o les groupes mono-, di- arylamino dans lesquels la partie aryle est éventuellement substituée par un
ou plusieurs substituants identiques ou différents, choisis parmi les atomes d'halogène ; les radicaux alkyle linéaire ou ramifié en C₁ à C₁₆, de préférence en C₁ à C₁₀ ; les radicaux alcoxy linéaire ou ramifié en C₁ à C₁₆, de préférence en C₁-C₁₀ ; les radicaux aryloxy éventuellement substitué ; mésyle (CH₃-SO₂-) ; amino ; amino mono ou disubstitué par un ou plusieurs groupements identiques ou non, choisis parmi les radicaux alkyle linéaires ou ramifiés en C₁-C₄, phényle, dans lequel le ou les substituants du groupe amino tel que les groupements alkyle peuvent former, conjointement avec l'atome d'azote dudit groupement amino auquel ils sont rattachés, un hétérocycle de préférence saturé, comprenant de 5 à 12 chaînons, de préférence de 5 à 7 chaînons, comprenant éventuellement un autre hétéroatome, choisi parmi l'oxygène, l'azote et le soufre ; ledit hétérocycle pouvant éventuellement être substitué, ledit hétérocycle pouvant éventuellement être condensé avec un cycle aromatique de préférence à 6 chaînons, ledit cycle aromatique étant éventuellement substitué.

De préférence encore, R₃, R₄, R₅, R₆, R₇, R₉ identiques ou différents représentent un atome d'hydrogène, un groupe amino, ou un groupe amino substitué par un ou plusieurs groupements identiques ou non, choisis parmi les radicaux alkyle linéaires ou ramifiés en C₁-C₄, phényle.

De préférence, l'un des radicaux R₃, R₄, R₅ représente un groupement amino ou amino substitué et/ou l'un des radicaux R₆, R₇, R₉ représente un groupement amino ou amino substitué.

Les positions préférées de R₃, R₄, R₅ sont les positions 6, 7, 8.

De préférence, un groupe amino ou amino substitué se trouve en position 7.

Les positions préférées de R₆, R₇ sont les positions 1 et 3.

De préférence, l'un des R₃, R₄, R₅ est un atome d'hydrogène.

De préférence l'un des R₆, R₇ est un atome d'hydrogène.

L₁ est de préférence une chaîne alkyle, linéaire ou ramifiée en C₁-C₁₆ éventuellement interrompue par au moins un hétéroatome choisi parmi l'azote, l'oxygène et le soufre, et/ou par au moins un groupement comprenant au moins un hétéroatome choisi parmi l'azote, l'oxygène et le soufre.

An représente de préférence un anion ou un mélange d'anions, organiques ou minéraux par exemple choisi parmi un halogénure tel que chlorure, bromure, fluorure, iodure ; un hydroxyde ; un sulfate ; un hydrogénosulfate ; un alkylsulfate pour lequel la partie alkyle, linéaire ou ramifiée, est en C₁-C₆, comme l'ion méthylsulfate, éthylsulfate ; les carbonates et hydrogénocarbonates ; des sels d'acides carboxyliques tels que le formiate, l'acétate, le citrate, le tartrate, l'oxalate ; les alkylsulfonates pour lesquels la partie alkyle, linéaire ou ramifiée, est en C₁-C₆ comme l'ion méthylsulfonate ; les arylsulfonates pour lesquels la partie aryle, de préférence phényle, est éventuellement substituée par un ou plusieurs radicaux alkyle en C₁-C₄ tel que par exemple le 4-toluylsulfonate ; les alkylsulfonyles tel que le mésylate.

Les composés de formule (I) peuvent être définis comme étant des colorants directs de type sulfonamidoxanthéniques, sulfonamide de xanthène, ou encore comme des colorants directs xanthéniques portant des groupements sulfonamides.

On fournit ci-après, à titre d'illustration, une liste donnant les formules de composés de formule (I) qui peuvent être utilisés dans le cadre de la présente invention

| | | |
|---|---|---|
| 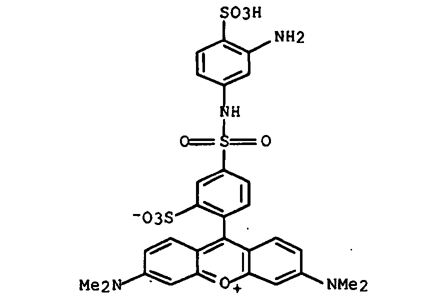 | 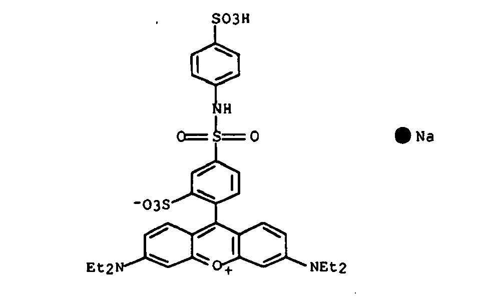 | |
| Sel interne de Xanthylium, 9-[4-[[(3-amino-4-sulfophenyl)nmino]sulfonyl]-2-sulfophenyl]- 3,6-bis(dimethylamino) | Sel interne de Xanthylium, 3,6-bis(diethylamino)-9-[2-sulfo-4-[[(4-sulfophenyl)amino]sulfonyl]phenyl]-, | |
| 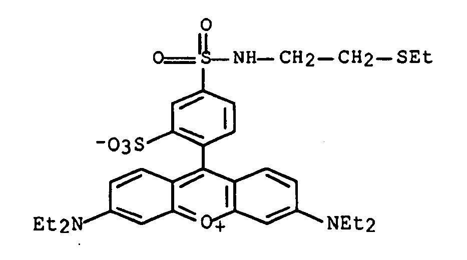 | 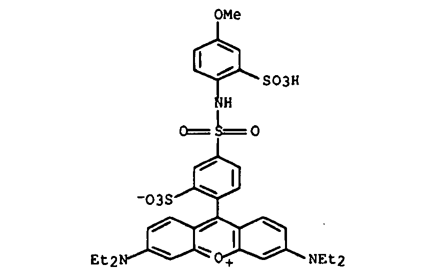 | |
| Sel interne de Xanthylium, 3,6-bis(diethylamino)-9-[4-[[[2-(ethylthio)ethyl]amino]sulfonyl]-2-sulfophenyl]-, | Sel interne de Xanthylium, 3,6-bis(diethylamino)-9-[4-[[(4-methoxy-2-sulfophenyl)amino]sulfonyl]-2-sulfophenyl] | |
| 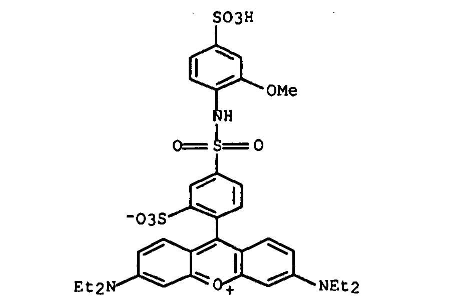 | 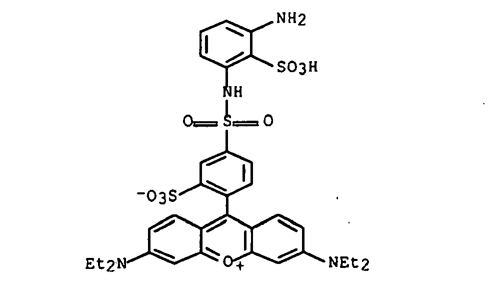 | |
| Sel interne de Xanthylium, 3,6-bis(diethylamino)-9-[4-[[(2-methoxy-4-sulfophenyl)amino]sulfonyl]-2-sulfophenyl] | Sel interne de Xanthylium, 9-[4-[((3-amino-2-sulfophenyl)amino]sulfonyl]-2-sulfophenyl]-3,6-bis(diethylamino)-, | |
| 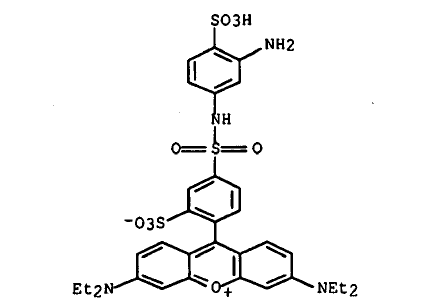 | 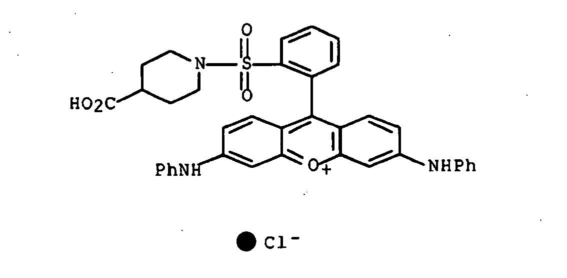 | |
| Sel interne de Xanthylium, 9-[4-[[(3-amino-4-sulfophenyl)amino]sulfonyl]-2-sulfophenyl]- 3,6-bis(diethylamino) | Xanthylium, 9-[2-[(4-carboxy-1-piperidinyl)sulfonyl]phenyl]-3,6-bis(phenylamino)-, Chlorure de | |
| 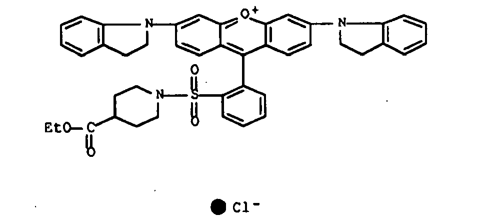 | 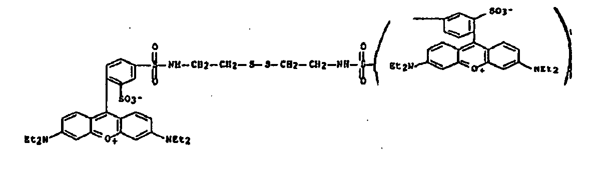 | |
| Xanthylium, 3,6-bis(2,3-dihydro-1H-indol-1-yl)-9-[2-[[4-(ethoxycarbonyl)-1-piperidinyl]sulfonyl]phenyl]-, Chlorure de | Sel interne de Xanthylium, 9,9'-[dithiobis[2,1-ethanediyliminosulfonyl(2-sulfo-4,1-phenylene)]]bis[3,6-bis(diethylamino) | |
| 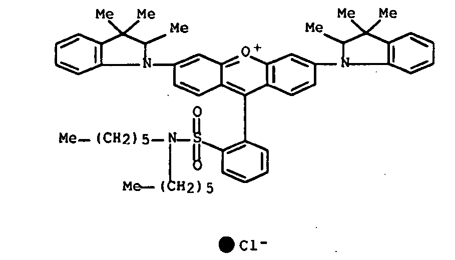 | 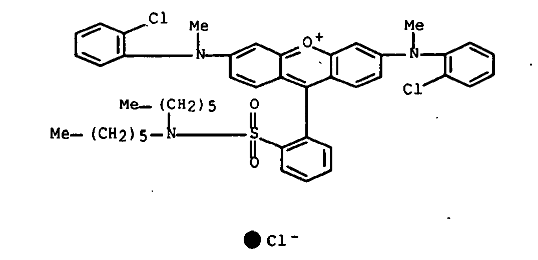 | |
| Xanthylium, 9-[2-[(dihexylamino)sulfonyl]phenyl]-3,6-bis(2,3-dihydro-2,3,3-trimethyl-1H-indol-1-yl)-, Chlorure de | Xanthylium, 3,6-bis[(2-chlorophenyl)methylamino]-9-[2-[(dihexylamino)sulfonyl]phenyl]-, Chlorure de | |
| 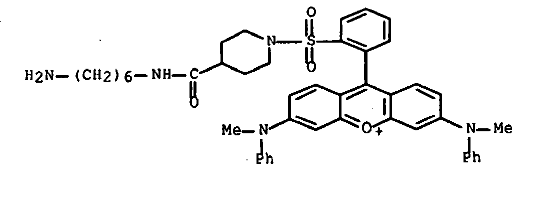 | 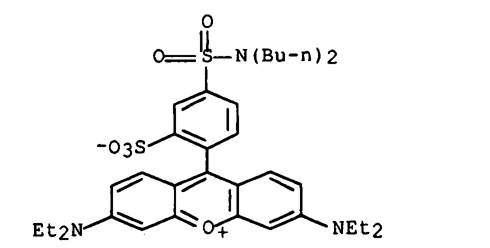 | |
| Xanthylium, 9-[2-[[4-[[(6-aminohexyl)amino]carbonyl]-1-piperidinyl]sulfonyl]phenyl]-3,6-bis(methylphenylamino) | Sel interne de Xanthylium, 9-[4-[(dibutylamino)sulfonyl]-2-sulfophenyl]-3,6-bis(diethylamino) | |
| 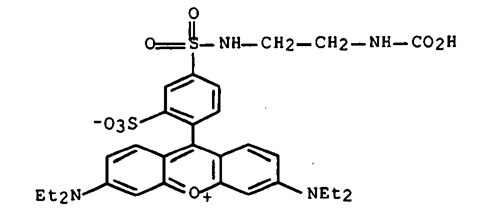 | 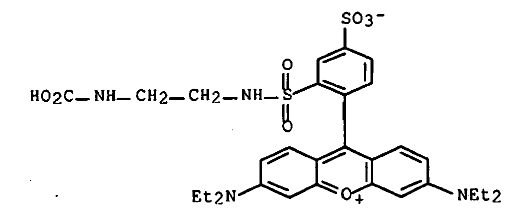 | |
| Sel interne de Xanthylium, 9-[4-[[[2-(carboxyamino)ethyl]aminojsulfonyl]-2-sulfophenyl]- 3,6-bis(diethylamino) | Sel interne de Xanthylium, 9-[2-[[[2-(carboxyamino)ethyl]amino]sulfonyl]-4-sulfophenyl]- 3,6-bis(diethylamino) | |
| 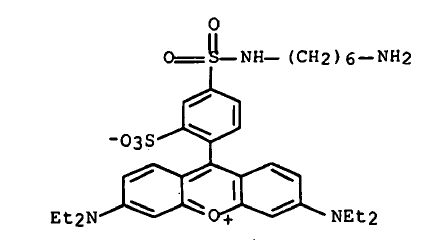 | 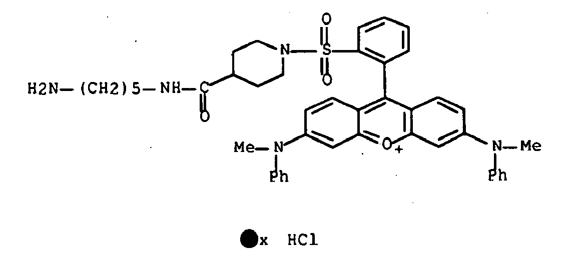 | |
| Sel interne de Xanthylium, 9-[4-[[(6-aminohexyl)amino]sulfonyl]-2-sulfophenyl]-3,6-bis(diethylamino) | Xanthylium, 9-[2-[[4-[[(5-aminopentyl)amino]carbonyl]-1-piperidinyl]sulfonyl]phenyl]-3,6-bis(methylphenylamino)-, Chlorhydrate | |
| 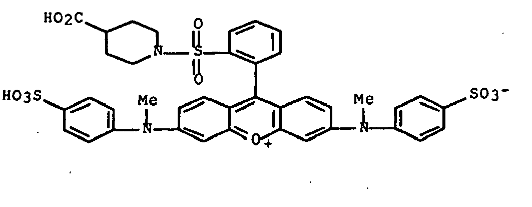 | 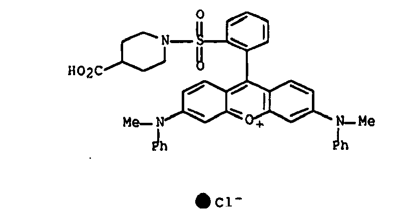 | |
| Sel interne de Xanthylium, 9-[2-[(4-carboxy-1-piperidinyl)sulfonyl]phenyl]-3,6-bis[methyl(4-sulfophenyl)amino] | Xanthylium, 9-[2-[(4-carboxy-1-piperidinyl)sulfonyl]phenyl]-3,6-bis(methylphenylamino)-, Chlorure de | |
| 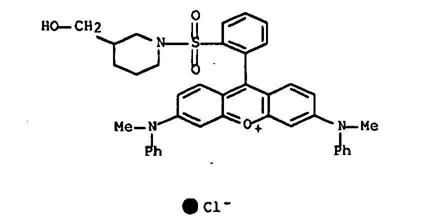 | 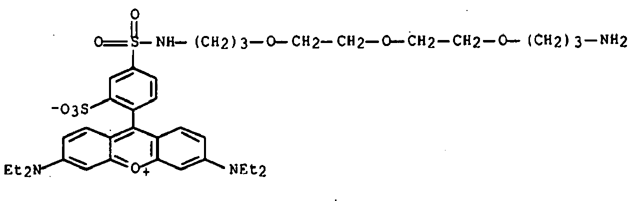 | |
| Xanthylium, 9-[2-[[3-(hydroxymethyl)-1-piperidinyl]sulfonyl]phenyl]-3,6-bis(methylphenylamino)-, Chlorure de | Sel interne de Xanthylium, 9-[4-[[[3-[2-[2-(3-aminopropoxy)ethoxy]ethoxy]propyl]amino]sulfonyl]-2-sulfophenyl]-3,6-bis(diethylamino) | |
| 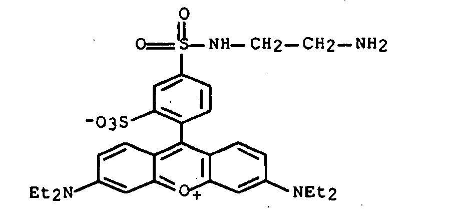 | 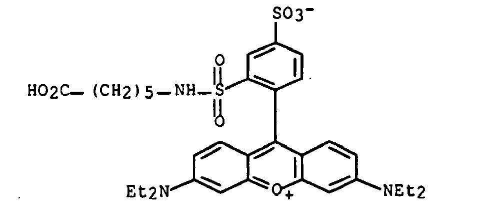 | |
| Sel interne de Xanthylium, 9-[4-[[(2-aminoethyl)aminolsulfonyl]-2-sulfophenyl]-3,6-bis(diethylamino) | Sel interne de Xanthylium, 9-[2-[[(5-carboxypentyl)amino]sulfonyl]-4-sulfophenyl]-3,6-bis(diethylamino) | |
| 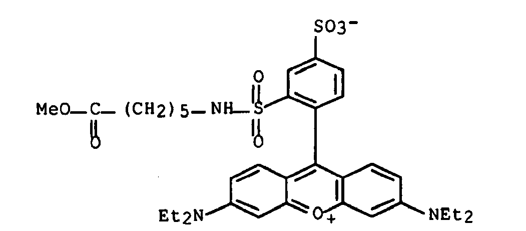 | 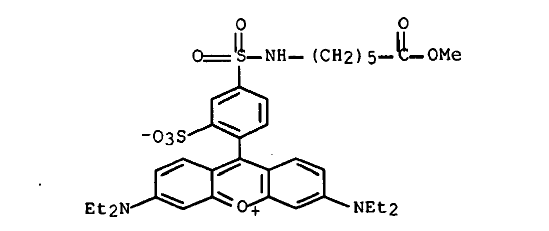 | |
| Sel interne de Xanthylium, 3,6-bis(diethylamino)-9-[2-[[(6-methoxy-6-oxohexyl)amino]sulfonyl]-4-sulfophenyl] | Sel interne de Xanthylium, 3,6-bis(diethylamino)-9-[4-[[(6-methoxy-6-oxohexyl)amino]sulfonyl]-2-sulfophenyl] | |
| 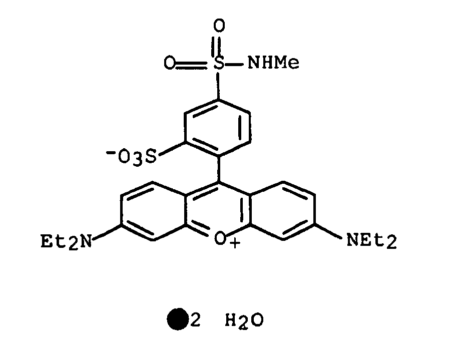 | 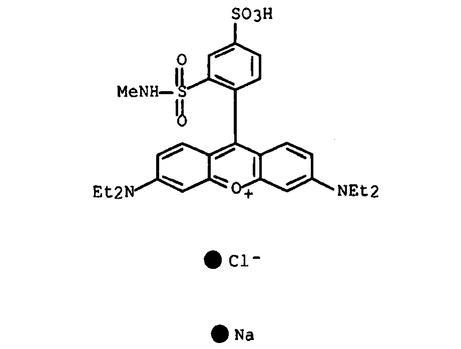 | |
| Sel interne dihydrate de Xanthylium, 3,6-bis(diethylamino)-9-[4-[(methylamino)sulfonyl]-2-sulfophenyl] | Xanthylium, 3,6-bis(diethylamino)-9-[2-[(methylamino)sulfonyl]-4-sulfophenyl]-, Chlorure de, monosel de sodium | |
| 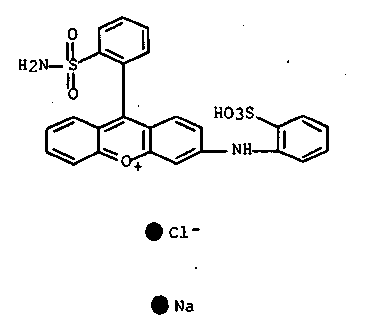 | 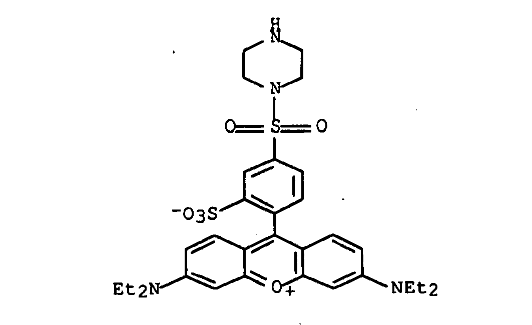 | |
| Xanthylium, 9-[2-(aminosulfonyl)phenyl]-3-[(2-sulfophenyl)amino]-, Chlorure de sel de sodium | Sel interne de Xanthylium, 3,6-bis(diethylamino)-9-[4-(1-piperazinylsulfonyl)-2-sulfophenyl] | |
| 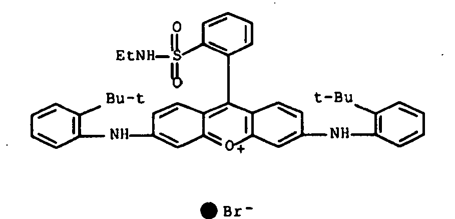 | 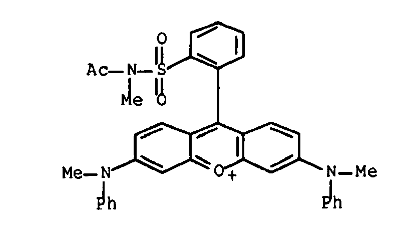 | |
| Xanthylium, 3,6-bis[[2-(1,1-dimethylethyl)phenyl]amino]-9-[2-[(ethylamino)sulfonyl]phenyl]-, Bromure de | Xanthylium, 9-[2-[(acetylmethylamino)sulfonyl]phenyl]-3,6-bis(methylphenylamino)-, hexafluorophosphate(1-) | |
| 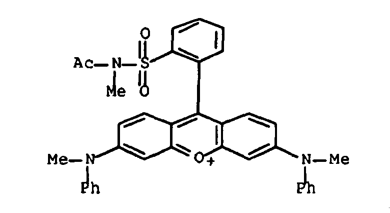 | 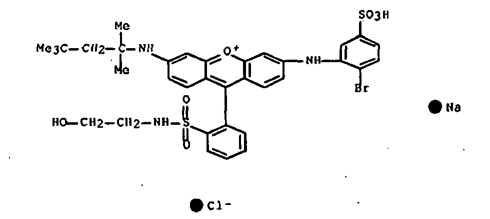 | |
| Xanthylium, 9-[2-[(acetylmethylamino)sulfonyl]phenyl]-3,6-bis(methylphenylamino)- | Xanthylium, 3-[(2-bromo-5-sulfophenyl)amino]-9-[2-[[(2- hydroxyethyl)amino]sulfonyl]phenyl]-6-[(1,1,3,3-tetramethylbutyl)amino]-, Chlorure de, monosel de sodium | |
| 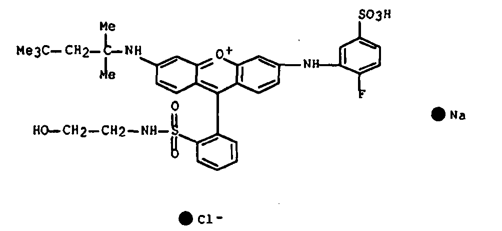 | 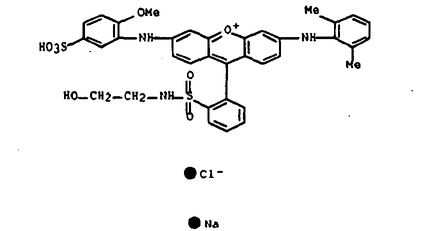 | |
| Xanthylium, 3-[(2-fluoro-5-sulfophenyl)amino]-9-[2-[[(2-hydroxyethyl)amino]sulfonyl]phenyl]-6-[(1,1,3,3-tetramethylbutyl)amino]-, Chlorure de, monosel de sodium | Xanthylium, 3-[(2,6-dimethylphenyl)amino]-9-[2-[[(2-hydroxyethyl)nmino]sulfonyl]phenyl]-6-[(2-methoxy-5-sulfophenyl)amino]-, Chlorure de, monosel de sodium | |
| 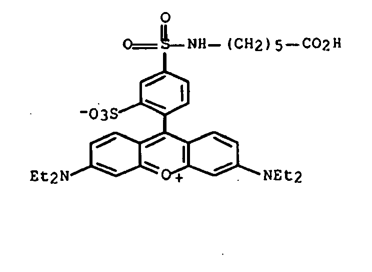 | 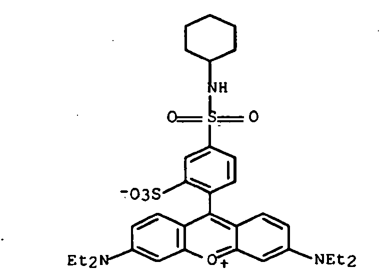 | |
| Sel interne de Xanthylium, 9-[4-[[(5-carboxypentyl)amino]sulfonyl]-2-sulfophenyl]-3,6-bis(diethylamino) | Sel interne de Xanthylium, 9-[4-[(cyclohexylamino)sulfonyl]-2-sulfophenyl]-3,6-bis(diethylamino) | |
| 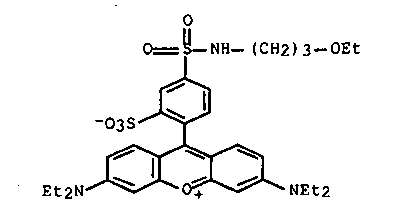 | 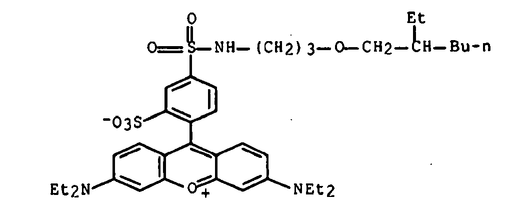 | |
| Sel interne de Xanthylium, 3,6-bis(diethylamino)-9-[4-[[(3-ethoxypropyl)amino]sulfonyl]-2-sulfophenyl] | Sel interne de Xanthylium, 3,6-bis(diethylamino)-9-[4-[[[3-[(2-ethylhexyl)oxy]propyl]amino]sulfonyl]-2-sulfophenyl] | |
| 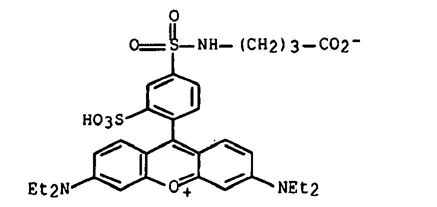 | 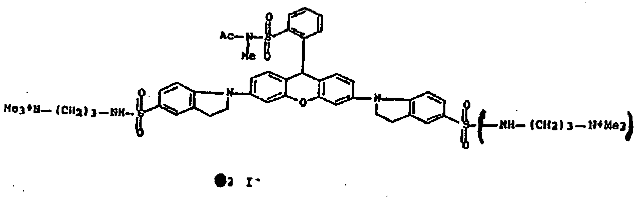 | |
| Sel interne de Xanthylium, 9-[4-[[(3-carboxypropyl)amino]sulfonyl]-2-sulfophenyl]-3,6-bis(diethylamino) | 1-Propanaminium, 3,3'-[[9-[2-[(acetylmethylamino)sulfonyl]phenyl]-9H-xanthene-3,6-diyl]bis[(2,3-dihydro-1H-indole-1,5-diyl)sulfonylimino]]bis[N,N,N-trimethyl-, diiodure | |
| 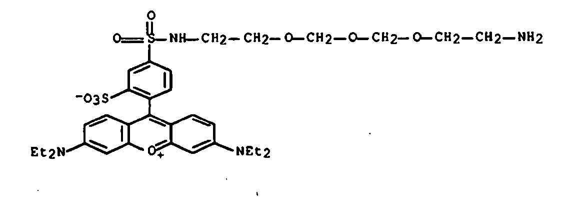 | 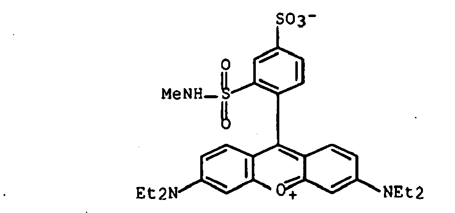 | |
| Sel interne de Xanthylium, 9-[4-[[[2-[[(2-aminoethoxy)methoxy]methoxy]ethyl]amino]sulfonyl]-2-sulfophenyl]-3,6-bis(diethylamino) | Sel interne de Xanthylium, 3,6-bis(diethylamino)-9-[2-[(methylamino)sulfonyl]-4-sulfophenyl] | |
| 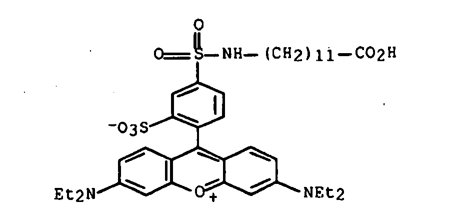 | 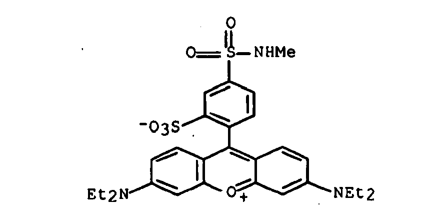 | |
| Sel interne de Xanthylium, 9-[4-[[(11-carboxyundecyl)amino]sulfonyl]-2-sulfophenyl]-3,6-bis(diethylamino) | Sel interne de Xanthylium, 3,6-bis(diethylamino)-9-[4-[(methylamino)sulfonyl]-2-sulfophenyl] | |
| 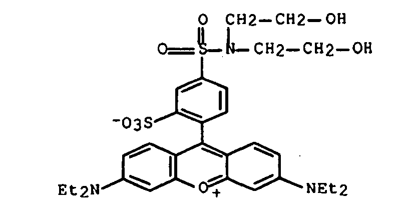 | 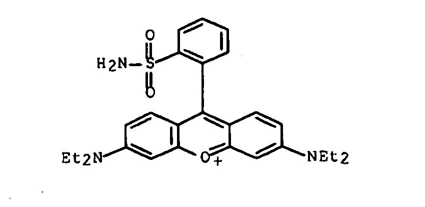 | |
| [9-[4-[Bis(2-hydroxyethyl)sulfamoyl]-2-sulfophenyl]-6-(diethylamino)-3H-xanthen-3-ylidene]diethylammonium hydroxide, sel interne | Xanthylium, 9-[2-(aminosulfonyl)phenyl]-3,6-bis(diethylamino)-, sel avec l'acide 4-methylbenzenesulfonique (1:1) | |
| 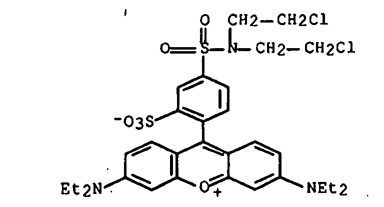 | 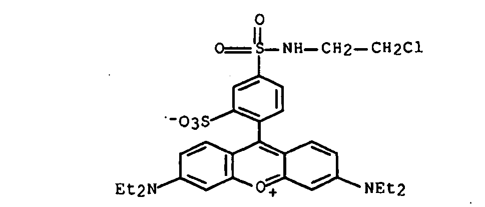 | |
| Sel interne de [9-[4-[Bis(2-chloroethyl)sulfamoyl]-2-sulfophenyl]-6-(diethylamino)-3H-xanthen-3-ylidene]diethylammonium hydroxide | Sel interne de [9-[4-[(2-Chloroethyl)sulfamoyl]-2-sulfophenyl]-6-(diethylamino)-3H-xanthen-3-ylidene]diethylammonium hydroxide | |
| 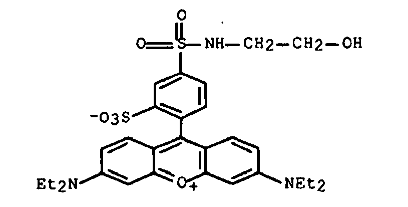 | 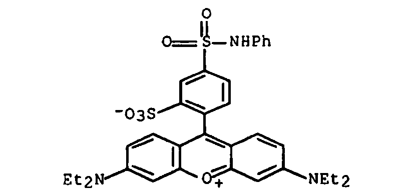 | |
| Sel interne de Xanthylium, 3,6-bis(diethylamino)-9-[4-[[(2-hydroxyethyl)amino]sulfonyl]-2-sulfophenyl] | Sel interne de [6-(Diethylamino)-9-[4-(phenylsulfamoyl)-2-sulfophenyl]-3H-xanthen-3-ylidene]diethylammonium hydroxide | |
| 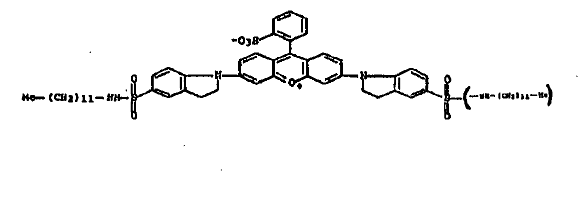 | 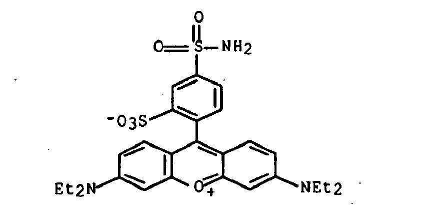 | |
| Sel interne de Xanthylium, 3,6-bis[5-[(dodecylamino)sulfonyl]-2,3-dihydro-1H-indol-1-yl]- 9-(2-sulfophenyl) | Sel interne de [6-(Diethylaminc)-9-(4-sulfamoyl-2-sulfophenyl)-3H-xanthen-3-ylidene]diethylammonium hydroxide | |
| 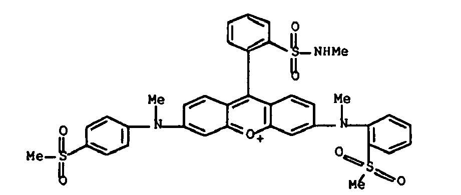 | 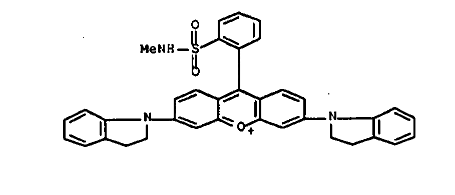 | |
| Xanthylium, 9-[2-[(methylamino)sulfonyl]phenyl]-3-[methyl[2- (methylsulfonyl)phenyl]amino]-6-[methyl[4-(methylsulfonyl)phenyl]amino]- | Xanthylium, 3,6-bis(2,3-dihydro-1H-indol-1-yl)-9-[2-[(methylamino)sulfonyl]phenyl]- | |
| 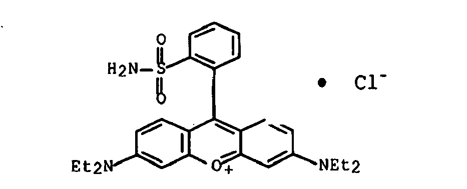 | 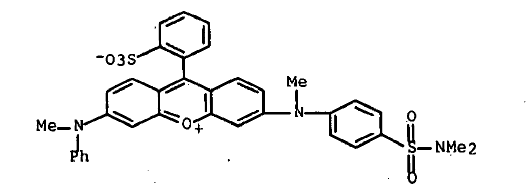 | |
| Xanthylium, 9-[2-(aminosulfonyl)phenyl]-3,6-bis(diethylamino)-chlorure | Sel interne de Xanthylium, 3-[[4-[(dimethylamino)sulfonyl]phenyl]methylamino]-6-(methylphenylamino)-9-(2-sulfophenyl) | |
| | | |
| 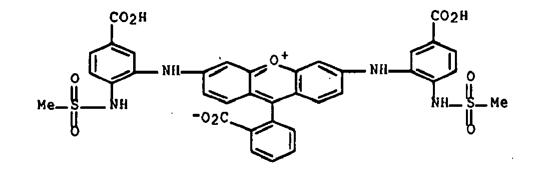 | 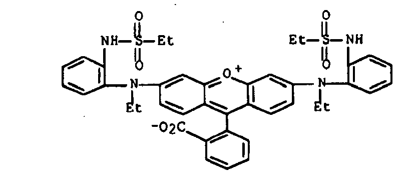 | |
| Sel interne de Xanthylium, 3,6-bis[[5-carboxy-2-[(methylsulfonyl)amino]phenyl]amino]-9-(2-carboxyphenyl) | Sel interne de Xanthylium, 9-(2-carboxyphenyl)-3,6-bis[ethyl[2-[(ethylsulfonyl)amino]phenyl]amino] | |
| 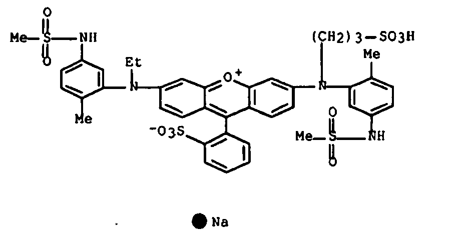 | 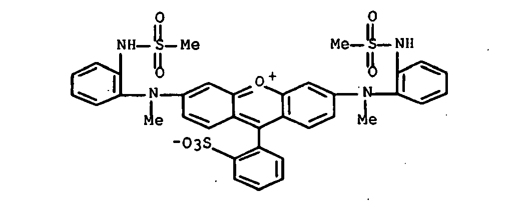 | |
| Sel interne de Xanthylium, 3-[ethyl[2-methyl-5-[(methylsulfonyl)amino]phenyl]amino]-6-[[2-methyl-5-[(methylsulfonyl)amino]phenyl](3-sulfopropyl)amino]-9-(2-sulfophenyl), monosel de sodium | Sel interne de Xanthylium, 3,6-bis[methyl[2-[(methylsulfonyl)amino]phenyl]amino]-9-(2-sulfophenyl) | |
| 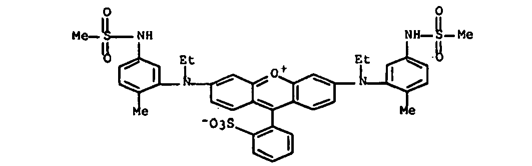 | 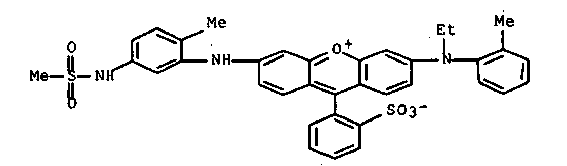 | |
| Sel interne de Xanthylium, 3,6-bis[ethyl[2-methyl-5-[(methylsulfonyl)amino]phenyl]amino]- 9-(2-sulfophenyl) | Sel interne de Xanthylium, 3-[ethyl(2-methylphenyl)amino]-6-[[2-methyl-5-[(methylsulfonyl)amino]phenyl]amino]-9-(2-sulfophenyl) | |
| 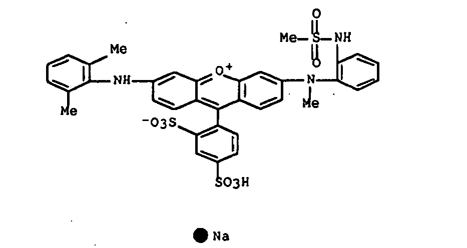 | 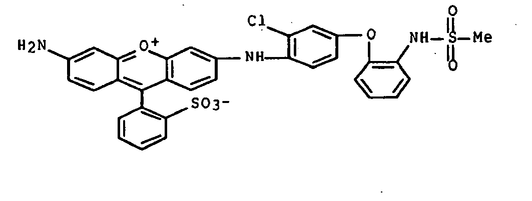 | |
| Sel interne de Xanthylium, 3-[(2,6-dimethylphenyl)amino]-9-(2,4-disulfophenyl)-6-[[2-[(methylsulfonyl)amino]phenyl]amino]-mono sel de sodium | Sel interne de Xanthylium, 3-amino-6-[[2-chloro-4-[2-[(methylsulfonyl)amino]phenoxy]phenyl]amino]-9-(2-sulfophenyl)-, | |
| 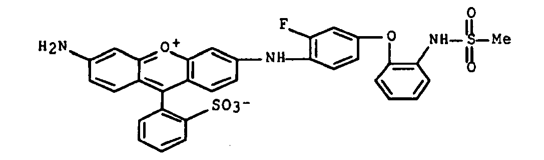 | 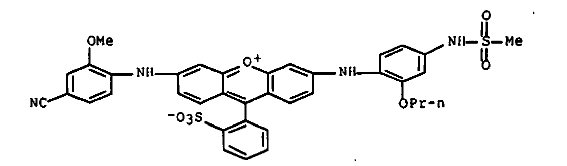 | |
| Sel interne de Xanthylium, 3-amino-6-[[2-fluoro-4-[2-[(methylsulfonyl)amino]phenoxy]phenyl]amino]-9-(2-sulfophenyl) | Sel interne de Xanthylium, 3-[(4-cyano-2-methoxyphenyl)amino]-6-[[4- [(methylsulfonyl)amino]-2-propoxyphenyl]amino]-9-(2-sulfophenyl) | |
| 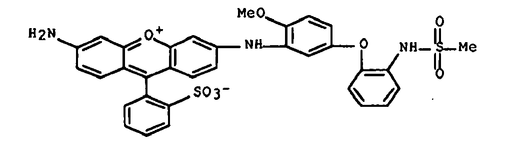 | 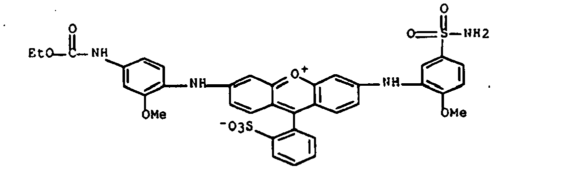 | |
| Sel interne de Xanthylium, 3-amino-6-[[2-methoxy-5-[2-[(methylsulfonyl)amino]phenoxy]phenyl]amino]-9-(2-sulfophenyl) | Sel interne de Xanthylium, 3-[[5-(aminosulfonyl)-2-methoxyphenyl]amino]-6-[[4- [(ethoxycarbonyl)amino]-2-methoxyphenyl]amino]-9-(2-sulfophenyl) | |
| 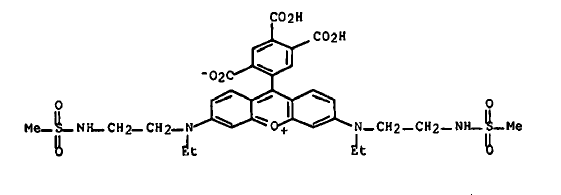 | 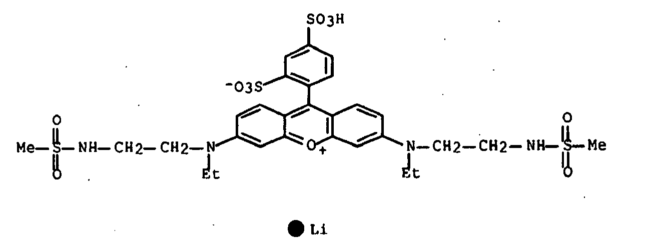 | |
| Sel interne de Xanthylium, 3,6-bis[ethyl[2-[(methylsulfonyl)amino]ethyl]amino]-9-(2,4,5-tricarboxyphenyl) | Sel interne de Xanthylium, 9-(2,4-disulfophenyl)-3,6-bis[ethyl[2- [(methylsulfonyl)amino]ethyl]amino] monosel de lithium | |
| 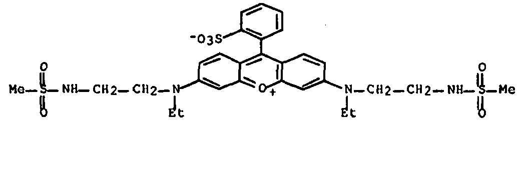 | 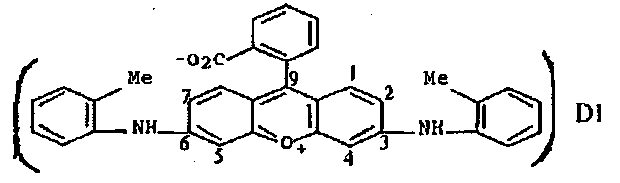 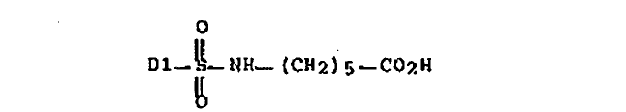 | |
| Sel interne de Xanthylium, 3,6-bis[ethyl[2-[(methylsulfonyl)amino]ethyl]amino]-9-(2-sulfophenyl) | Sel interne de Xanthylium, 3-[[[[(5-carboxypentyl)amino]sulf onyl]-2-methylphenyl]amino]-9- (2-carboxyphenyl)-6-[(2-methylphenyl)amino] | |
| 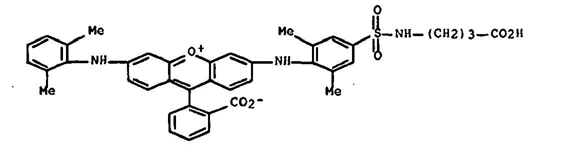 | 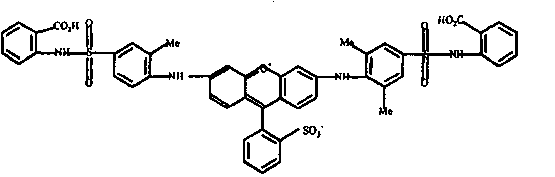 | |
| Sel interne de Xanthylium, 9-(2-carboxyphenyl)-3-[[4-[[(3-carboxypropyl)amino]sulfonyl]- 2,6-dimethylphenyl]amino]-6-[(2,6-dimethylphenyl)amino] | Sel interne de Xanthylium, 3,6-bis[[4-[[(2-carboxyphenyl)amino]sulfonyl]-2,6-dimethylphenyl]amino]-9-(2-sulfophenyl) | |
| 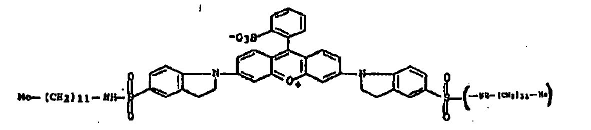 | 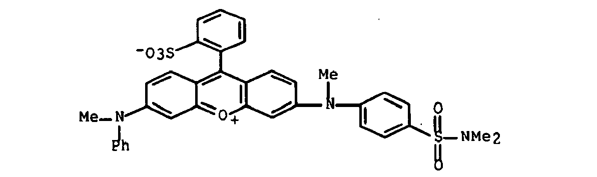 | |
| Sel interne de Xanthylium, 3,6-bis[5-[(dodecylamino)sulfonyl]-2,3-dihydro-1H-indol-1-yl]- 9-(2 sulfophenyl) | Sel interne de Xanthylium, 3-[[4-[(dimethylamino)sulfonyl]phenyl]methylamino]-6-(methylphenylamino)-9-(2-sulfophenyl) | |
| 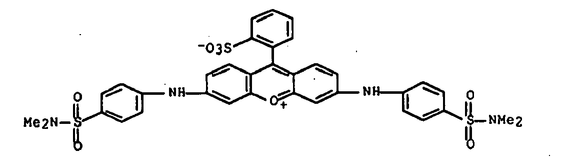 | 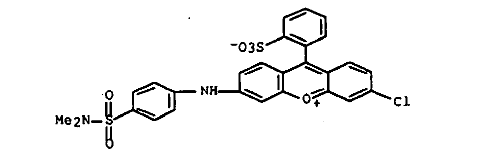 | |
| Sel interne de Xanthylium, 3,6-bis[[4-[(dimethylamino)sulfonyl]phenyl]amino]-9-(2-sulfophenyl) | Sel interne de Xanthylium, 3-chloro-6-[[4-[(dimethylamino)sulfonyl]phenyl]amino]-9-(2-sulfophenyl) | |
| 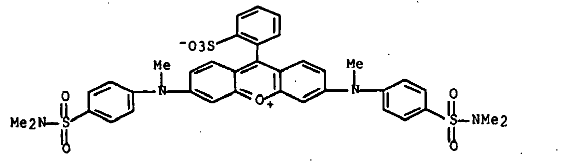 | 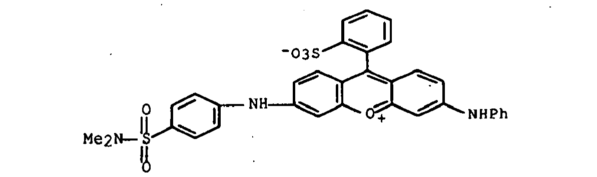 | |
| Sel interne de Xanthylium, 3,6-bis[[4-[(dimethylamino)sulfonyl]phenyl]methylamino]-9-(2-sulfophenyl) | Sel interne de Xanthylium, 3-[[4-[(dimethylamino)sulfonyl]phenyl]amino]-6-(phenylamino)-9-(2-sulfophenyl) | |
| 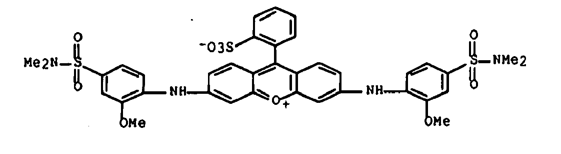 | 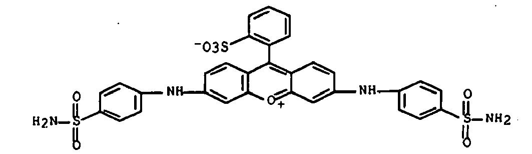 | |
| Sel interne de Xanthylium, 3,6-bis[[4-[(dimethylamino)sulfonyl]-2-methoxyphenyl]amino]-9- (2-sulfophenyl) | Sel interne de Xanthylium, 3,6-bis[[4-(aminosulfonyl)phenyl]amino]-9-(2-sulfophenyl) | |
| 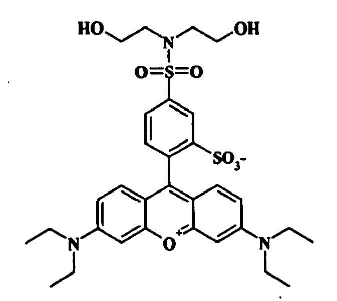 | 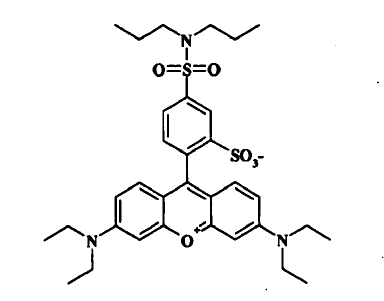 | 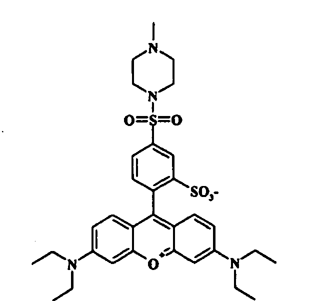 |
| 9-{4-[Bis-(2-hydroxy-ethyl)-sulfamoyl]-2-sulfo-phenyl}-3,6-bis-diethylamino-xanthenylium | 3,6-Bis-diethylamino-9-(4-dipropylsulfamoyl-2-sulfo-phenyl)-xanthenylium | 3,6-Bis-diethylamino-9-[4-(4-methyl-piperazine-1-sulfonyl)-2-sulfo-phenyl]-xanthenylium |
| 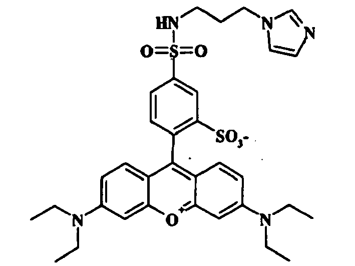 | 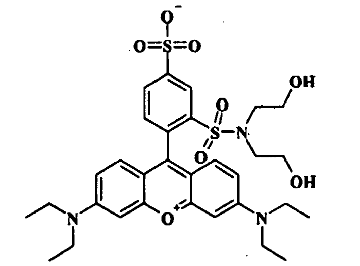 | 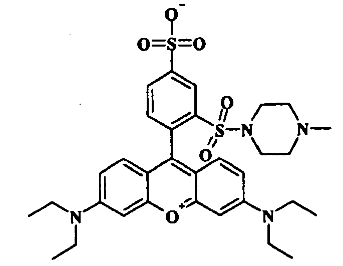 |
| 3,6-Bis-diethylamino-9-[4-(3-imidazol-1-yl-propylsulfamoyl)-2-sulfo-phenyl] -xanthenylium | 9-{2-[Bis-(2-hydroxy-ethyl)-sulfamoyl]-4-sulfo-phenyl}-3,6-bis-diethylamino- xanthenylium | 3,6-Bis-diethylamino-9-[2-(4-methyl-piperazine-1-sulfonyl)-4-sulfo-phenyl]-xanthenylium |

Il s'avère que les compositions de l'invention permettent, de manière surprenante, d'obtenir des colorations intenses.

Les compositions selon l'invention permettent d'obtenir des reflets variés chromatiques ou sombres, très puissants, peu sélectifs et tenaces.

Ainsi, les colorants (I) inclus dans les compositions de l'invention permettent d'obtenir toutes les nuances du vert au bleu, en passant par les rouges.

Les colorations obtenues avec les compositions de l'invention sont tenaces, stables, résistantes, vis-à-vis des intempéries, du lavage, de la transpiration, des frottements et des traitements ultérieurs, tels que l'ondulation permanente.

Ces colorations sont, en particulier, tenaces à la lumière.

Le ou les composés de formules (I) ci-dessus représentent généralement de 0,0001 à 10 % en poids du poids total de la composition de teinture, de préférence de 0,005 à 10 % en poids et, de préférence encore, de 0,01 à 6 % de poids du poids total de la composition de teinture selon l'invention.

La composition tinctoriale conforme à l'invention peut en outre contenir un ou plusieurs colorant(s) direct(s) différent(s) des composés de formule (I). Le ou ces colorant(s) direct(s) utile(s) selon l'invention sont par exemple choisis parmi les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques les colorants directs naturels, et les colorants directs xanthéniques différents des composés de formule (I).

Parmi les colorants directs benzéniques, on peut citer de manière non limitative les composés suivants :
- 1,4-diamino-2-nitrobenzène ;
- 1-amino-2 nitro-4-β- hydroxyéthylaminobenzène ;
- 1-amino-2 nitro-4-bis(β-hydroxyéthyl)-aminobenzène ;
- 1,4-Bis(β-hydroxyéthylamino)-2-nitrobenzène ;
- 1-β-hydroxyéthylamino-2-nitro-4-bis-(β-hydroxyéthylamino)-benzène ;
- 1-β-hydroxyéthylamino-2-nitro-4-aminobenzène ;
- 1-β-hydroxyéthylamino-2-nitro-4-(éthyl) (β-hydroxyéthyl)-aminobenzène ;
- 1-amino-3-méthyl-4-β-hydroxyéthylamino-6-nitrobenzène ;
- 1-amino-2-nitro-4-β-hydroxyéthylamino-5-chlorobenzène ;
- 1,2-Diamino-4-nitrobenzène ;
- 1-amino-2-β-hydroxyéthylamino-5-nitrobenzène ;
- 1,2-Bis-(β-hydroxyéthylamino)-4-nitrobenzène ;
- 1-amino-2-tris-(hydroxyméthyl)-méthylamino-5-nitrobenzène ;
- 1-Hydroxy-2-amino-5-nitrobenzène ;
- 1-Hydroxy-2-amino-4-nitrobenzène ;
- 1-Hydroxy-3-nitro-4-aminobenzène ;
- 1-Hydroxy-2-amino-4,6-dinitrobenzène ;
- 1-β-hydroxyéthyloxy-2-β-hydroxyéthylamino-5-nitrobenzène ;
- 1-Méthoxy-2-β-hydroxyéthylamino-5-nitrobenzène ;
- 1-β-hydroxyéthyloxy-3-méthylamino-4-nitrobenzène ;
- 1-β,γ-dihydroxypropyloxy-3-méthylamino-4-nitrobenzène ;
- 1-β-hydroxyéthylamino-4-β,γ-dihydroxypropyloxy-2-nitrobenzène ;
- 1-β,γ-dihydroxypropylamino-4-trifluorométhyl-2-nitrobenzène ;
- 1-β-hydroxyéthylamino-9-trifluorcanéthyl-2-nitrobenzène ;
- 1-β-hydroxyéthylamino-3-méthyl-2-nitrobenzène ;
- 1-β-aminoéthylamino-5-méthoxy-2-nitrobenzène ;
- 1-Hydroxy-2-chloro-6-éthylamino-4-nitrobenzène ;
- 1-Hydroxy-2-chloro-6-amino-4-nitrobenzène ;
- 1-Hydroxy-6-bis-(β-hydroxyéthyl)-amino-3-nitrobenzène :
- 1-β-hydroxyéthylamino-2-nitrobenzène ;
- 1-Hydroxy-4-β-hydroxyéthylamino-3-nitrobenzène.

Parmi les colorants directs azoïques, on peut citer les colorants azoïques cationiques décrits dans les demandes de brevets WO-A-95/15144, WO-A-95/01772, EP-A-714954 et WO-A-01/66646.

Parmi ces composés, on peut tout particulièrement citer les colorants suivants :
- chlorure de 1,3-diméthyl-2-[[4-(diméthylamino)phényl]azo]-1H-Imidazolium ;
- chlorure de 1,3-diméthyl-2-[(4-aminophényl)azo]-1H-Imidazolium ;
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3^{e} édition :
- Disperse Red 17 ;
- Acid Yellow 9 ;
- Acid Black 1 ;
- Basic Red 22 ;
- Basic Red 76 ;
- Basic Yellow 57 ;
- Basic Brown 16 ;
- Acid Yellow 36 ;
- Acid Orange 7 ;
- Acid Red 33 ;
- Acid Red 35 ;
- Basic Brown 17 ;
- Acid Yellow 23 ;
- Acid Orange 24 ;
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques, on peut citer les colorants suivants :
- Disperse Red 15 ;
- Solvent Violet 13 ;
- Acid Violet 43 ;
- Disperse Violet 1 ;
- Disperse Violet 4 ;
- Disperse Blue 1 ;
- Disperse Violet 8 ;
- Disperse Blue 3 ;
- Disperse Red 11 ;
- Acid Blue 62 ;
- Disperse Blue 7 ;
- Basic Blue 22 ;
- Disperse Violet 15 ;
- Basic Blue 99,
   ainsi que les composés suivants :

- 1-N-méthylmorpholiniumpropylamino-4-hydroxyanthraquinone ;
- 1-Aminopropylamino-4-méthylaminoanthraquinone ;
- 1-Aminopropylaminoanthraquinone ;
- 5-β-hydroxyéthyl-1,4-diaminoanthraquinone ;
- 2-Aminoéthylaminoanthraquinone ;
- 1, 4-Bis-(β,γ-dihydroxypropylamino) -anthraquinone.

Parmi les colorants aziniques, on peut citer les composés suivants :
- Basic Blue 17 ;
- Basic Red 2.

Parmi les colorants triarylméthaniques, on peut citer les composés suivants :
- Basic Green 1 ;
- Acid blue 9 ;
- Basic Violet 3 ;
- Basic Violet 14 ;
- Basic Blue 7 ;
- Acid Violet 49 ;
- Basic Blue 26 ;
- Acid Blue 7.

Parmi les colorants indoaminiques, on peut citer les composés suivants :
- 2-β-hydroxyéthlyamino-5-[bis-(β-4'-hydroxyéthyl)amino]anilino-1,4-benzoquinone ;
- 2-β-hydroxyéthylamino-5-(2'-méthoxy-4'-amino)anilino-1,4-benzoquinone ;
- 3-N(2'-Chloro-4'-hydroxy)phényl-acétylamino-6-méthoxy-1,4-benzoquinone imine ;
- 3-N(3'-Chloro-4'-méthylamino)phényl-uréido-6-méthyl-1,4-benzoquinone imine ;x
- 3-[4'-N-(Ethyl,carbamylméthyl)-amino]-phényl-uréido-6-méthyl-1,4-benzoquinone imine.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs, additionnels différents des composants de formules (I) représentent de préférence de 0,001 à 20% en poids environ du poids total de la composition prête à l'emploi et encore plus préférentiellement de 0,005 à 10% en poids environ.

La composition de la présente invention peut de plus contenir une ou plusieurs base(s) d'oxydation, et éventuellement un ou plusieurs coupleur(s) classiquement utilisés pour la coloration par oxydation.

A titre d'exemple de base d'oxydation, on peut citer les paraphénylènediamines, les bis-phénylalkylènediamines, les para-aminophénols, les ortho-aminophénols, les bases hétérocycliques et leurs sels d'addition.

Les coupleurs sont par exemple les coupleurs métaphénylènediamines, les coupleurs méta-aminophénols, les coupleurs métadiphénols, les coupleurs naphtaléniques, les coupleurs hétérocycliques et leurs sels d'addition.

Lorsqu'ils sont présents, le ou les base(s) d'oxydation et/ou le ou les coupleurs sont chacun généralement présents en une quantité comprise entre 0,001 et 10 % en poids environ du poids total de la composition tinctoriale, de préférence entre 0,005 et 6 %.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci-dessus sont en général présents en quantité comprise pour chacun d'eux entre 0,01 et 20 % en poids par rapport au poids de la composition.

De préférence, les compositions contiennent un solvant choisi parmi les alcanols en C₂-C₄ et les polyols de poids moléculaire inférieur à 1 000.

De préférence, les compositions contiennent au moins un agent tensioactif et/ou au moins un agent épaississant minéral ou organique.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, de préférence entre 5 et 11 environ, de préférence encore de 6 à 8,5.

Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants, on peut citer, à titre d'exemple, l'ammoniaque, les carbonates alcalins, les alcanolamines telles que les mono-, di-et triéthanolamines ainsi que leurs dérivés, les hydroxydes de sodium ou de potassium et les composés de formule (II) suivante : 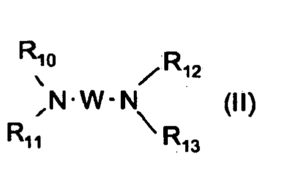 dans laquelle W est un reste propylène éventuellement substitué par un groupement hydroxyle ou un radical alkyle en C₁-C₄ ; R₁₀, R₁₁, R₁₂ et R₁₃, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels,
ou sous toute autre forme appropriée pour réaliser une teinture des fibres kératiniques, et notamment des cheveux humains.

L'invention a aussi pour objet un procédé de teinture directe des fibres kératiniques et, en particulier, des fibres kératiniques humaines, telles que les cheveux, qui comprend l'application d'au moins une composition tinctoriale contenant au moins un composé, colorant, de formule (I) telle que définie précédemment sur les fibres kératiniques. Après un temps de pause, les fibres kératiniques sont rincées laissant apparaître des fibres colorées. Le temps de pause est généralement compris entre 3 à 50 minutes environ, de préférence 5 à 30 minutes environ.

Par teinture ou coloration directe, on entend une teinture ou coloration effectuée sans agent oxydant organique ou minéral.

Avec les colorants de l'invention, on peut également réaliser de la coloration directe éclaircissante en l'absence de colorant d'oxydation et en présence d'un agent oxydant dans des conditions telles que celui-ci soit apte à éclaircir les fibres kératiniques par action sur les pigments initialement présents dans lesdites fibres.

Lorsque la composition tinctoriale comprend au moins un composé de formule (I) et au moins une base d'oxydation, et éventuellement un ou plusieurs coupleurs, la composition tinctoriale contient de préférence un oxydant.

L'invention a donc également pour objet un procédé de teinture des fibres kératiniques et, en particulier, des fibres kératiniques humaines, telles que les cheveux, dans lequel on applique sur lesdites fibres au moins une composition de teinture comprenant au moins un composé de formule (I) et éventuellement au moins une base d'oxydation et éventuellement un ou plusieurs coupleur(s), la couleur étant révélée à pH acide, neutre ou alcalin à l'aide d'un agent oxydant.

Les agents oxydants utilisables sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

L'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention. La composition oxydante peut également renfermer divers adjuvants utilisés classiquement dans les compositions pour la teinture des cheveux et tels que définis précédemment .

En cas de mélange avec la composition tinctoriale, le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11, mieux entre 6 et 8,5. Il peut être ajusté à la valeur désirée au moyen d'agents acidifiants ou alcalinisants habituellement utilisés en teinture des fibres kératiniques et tels que définis précédemment.

La composition qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

Un autre objet de l'invention est un dispositif à plusieurs compartiments ou « kit » de teinture, dans lequel un premier compartiment renferme une composition tinctoriale définie ci-dessus, comprenant soit au moins un composé de formule (I) et éventuellement au moins une base d'oxydation et éventuellement un ou plusieurs coupleurs, et un deuxième compartiment renferme une composition oxydante.

Un autre objet de l'invention est un dispositif à plusieurs compartiments ou « kit » de teinture, dans lequel un premier compartiment renferme une composition tinctoriale comprenant au moins un composé de formule (I), un second compartiment renferme une composition tinctoriale comprenant au moins une base d'oxydation et éventuellement au moins un coupleur, et un troisième compartiment renferme une composition oxydante.

Ce dispositif peut être équipé d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention.

### Exemples

On a préparé la composition de teinture suivante :

| **Colorant 1** | | 0,553 g |
|---|---|---|
| Diéthanolamide oléïque | | 3 g |
| Acide laurique | | 1 g |
| Monoéthyléther de l'éthylèneglycol | | 5 g |
| Hydroxyéthylcellulose | | 2 g |
| 2-amino-2-méthyl-1- propanol | q.s. pH | 9,5 |
| Eau déminéralisée | q.s. p | 100 g |

### Colorant 1 : 9-{4-[Bis-(2-hydroxy-éthyl)-sulfamoyl]-2-sulfo-phenyl}-3,6-bis-diéthylamino-xanthénylium

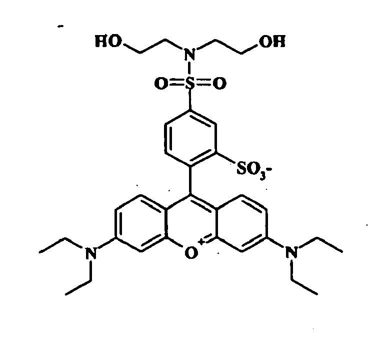

On a appliqué la composition ci-dessus sur des mèches de cheveux gris naturels ou permanentés à 90% de blancs et on a laissé poser pendant 30 minutes.

Après rinçage à l'eau courante et séchage, les cheveux ont été teints dans une coloration rose soutenue.

On obtient les mêmes résultats en utilisant les colorants suivants :

### Colorant 2 : 3,6-Bis-diéthylamino-9-(4-dipropylsulfamoyl-2-sulfo-phényl)-xanthénylium

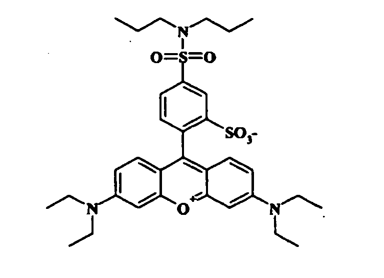

### Colorant 3 : 3,6-Bis-diéthylamino-9-[4-(4-méthylpipérazine-1-sulfonyl)-2-sulfo-phényl]- xanthénylium

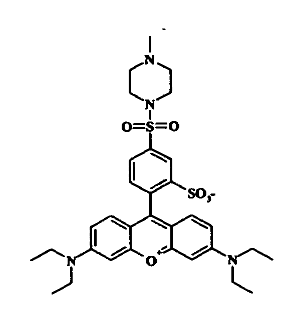

### Colorant 4 : 3,6-Bis-diéthylamino-9-[4-(3-imidazol-1-yl-propylsulfamoyl)-2-sulfo-phényl] - xanthénylium

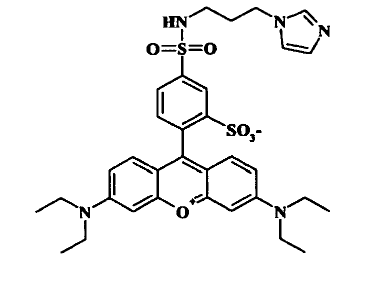

### Colorant 5 : 9-{2-[Bis-(2-hydroxy-éthyl)-sulfamoyl]-4-sulfo-phényl}-3,6-bis-diéthylamino- xanthénylium

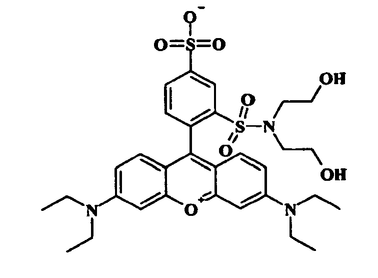

### Colorant 6 : 3,6-Bis-diéthylamino-9-[2-(4-méthylpipérazine-1-sulfonyl)-4-sulfo-phényl]- xanthénylium

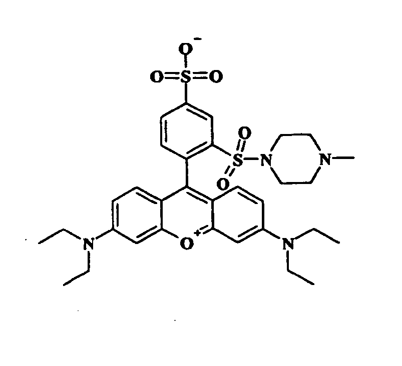

## Revendications

1. Composition pour la teinture des fibres kératiniques comprenant dans un milieu cosmétique approprié pour la teinture, au moins un colorant choisi parmi les composés répondant à la formule (I) suivante, leurs formes mésomères, et leurs oligomères : 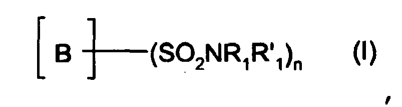 dans laquelle B représente : 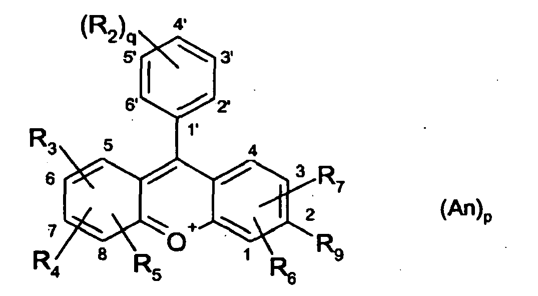 dans laquelle R₁, R'₁, identiques ou non, représentent :
o un atome d'hydrogène,
o un radical alkyle,
o un radical thioalkyle,
o un radical alcoxy,
o un radical amino alkyle,
o un radical trialkylammonioalkyle,
o un radical carboxy alkyle,
o un radical carboxy amino alkyle,
o un radical amino alcoxy polyalcoxy dans lequel les radicaux alcoxy du groupement polyalcoxy, identiques ou non, comprennent chacun 1 à 4 atomes de carbone,
o un radical alcoxycarbonyle,
o un radical alcoxycarbonylalkyle,
o un radical alcoxyalkyle,
o un radical acyle,
o un radical aryle, de préférence phényle,
o un radical arylalkyle, de préférence benzyle,
o un hétérocycle avec un atome d'azote, à 5 à 12 chaînons, de préférence 5 à 7 chaînons, saturé ou non, comprenant éventuellement au moins un autre hétéroatome choisi parmi l'oxygène, l'azote ou le soufre, ledit hétérocycle étant éventuellement substitué ;
R'₁ pouvant également représenter le groupement suivant : 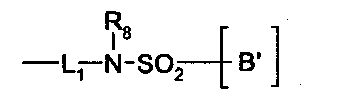
dans laquelle :
• R₈, représente un groupement choisi parmi ceux définissant R₁ et R'₁,
• L₁ représente une chaîne hydrocarbonée divalente comprenant de 1 à 16 atomes de carbone, éventuellement interrompue par au moins un hétéroatome choisi parmi l'oxygène, le soufre ou l'azote, et/ou par un groupement comprenant au moins un hétéroatome, tel que par exemple CO, SO₂,
• B' représente un groupement de formule (B) et est identique ou non à B ;
les radicaux R₁ et R'₁ forment éventuellement ensemble avec l'atome d'azote auquel ils sont rattachés, un hétérocycle, de préférence saturé, comprenant de 5 à 12 chaînons, de préférence de 5 à 7 chaînons, comprenant éventuellement un autre hétéroatome, choisi parmi l'oxygène, l'azote ou le soufre ; ledit hétérocycle étant éventuellement substitué ;
- R₂ représente un groupement choisi parmi l'atome d'hydrogène, les atomes d'halogène tels que F, Cl, Br et I, les radicaux alcoxy, les radicaux alkyle, le radical sulfonique, le radical carboxy ;
- R₃, R₄, R₅, R₆, R₇, R₉ représentent chacun indépendamment un groupement choisi parmi l'atome d'hydrogène ; les atomes d'halogène tels que F, Cl, Br et I ; les radicaux alcoxy ; les radicaux alkyle ; les groupes amino ; les groupes amino, mono ou disubstitués par un groupe choisi parmi le groupe benzyle, les groupes aryle, et les groupes alkyle, tels que les groupes monoaryl amino, diaryl amino, monoalkylamino, (dialkyl)amino dans lesquels les substituants du groupe amino tels que les radicaux alkyle, peuvent former ensemble avec l'atome d'azote auquel ils sont liés un hétérocycle, de préférence saturé de 5 à 12 chaînons, de préférence de 5 à 7 chaînons comprenant éventuellement au moins un autre hétéroatome choisi parmi l'azote, l'oxygène et le soufre, ledit hétérocycle pouvant être éventuellement substitué ; ou bien un ou les deux substituants du groupe amino tels que les radicaux alkyle peuvent former ensemble avec un substituant situé en position ortho du groupe amino mono ou disubstitué tel que monoalkylamino ou (dialkyl)amino un hétérocycle à 5 à 6 chaînons pouvant éventuellement comprendre en outre au moins un autre hétéroatome choisi parmi les atomes d'azote, d'oxygène et de soufre, ledit hétérocycle pouvant être éventuellement substitué ;
le ou les groupements -SO₂NR₁R'₁ et/ou - L₁NR₈SO₂- étant rattachés soit directement sur un ou plusieurs des cycles porteurs des substituants R₂ à R₉ ou par l'intermédiaire d'un ou plusieurs des substituants R₂ à R₉ ;
le coefficient n est un entier allant de 1 à 4, de préférence égal à 1 ;
le coefficient q est un entier allant de 1 à 3, de préférence égal à 1 ;
An représente un anion mono ou polyvalent, ou un mélange d'anions ;
le coefficient p est égal à 0 ou 1 ;
An et p étant tels que le composé soit électriquement neutre.

2. Composition de teinture selon la revendication 1, dans laquelle dans la formule (I) R₁, R'₁, identiques ou non, représentent :
o un atome d'hydrogène,
o un radical alkyle en C₁-C₁₆, linéaire ou ramifié, ou cyclique en C₃-C₁₆, éventuellement substitué par un ou plusieurs groupements identiques ou non, choisis parmi les groupements amino, sulfonique, et alcoxy,
o un radical hydroxyalkyle en C₁-C₁₆, linéaire, ramifié, ou cyclique en C₃-C₁₆,
o un radical thioalkyle en C₁-C₁₆, linéaire, ramifié, ou cyclique en C₃-C₁₆,
o un radical alcoxy en C₁-C₁₆, linéaire ou ramifié,
o un radical amino alkyle en C₁-C₁₆, linéaire, ou ramifié, ou cyclique en C₃-C₁₆,
o un radical trialkylammonioalkyle dans lequel les radicaux alkyle, identiques ou non, sont en C₁-C₁₆,
o un radical halogénoalkyle linéaire, ou ramifié dans lequel le radical alkyle linéaire ou ramifié comprend 1 à 16 atomes de carbone,
o un radical carboxy alkyle en C₁-C₁₆, linéaire, ramifié ou cyclique en C₃-C₁₆,
o un radical carboxy amino alkyle en C₁-C₁₆, linéaire ou ramifié, ou cyclique en C₃-C₁₆,
o un radical amino alcoxy polyalcoxy dans lequel les radicaux alcoxy, identiques ou non, linéaires ou ramifiés comprennent 1 à 4 atomes de carbone,
o un radical alcoxycarbonyle dans lequel le radical alcoxy linéaire, ou ramifié comprend 1 à 16 atomes de carbone,
o un radical alcoxycarbonylalkyle dans lequel le radical alcoxy linéaire, ou ramifié comprend 1 à 16 atomes de carbone et le radical alkyle linéaire ou ramifié comprend 1 à 16 atomes de carbone,
o un radical alcoxyalkyle dans lequel le radical alcoxy linéaire, ou ramifié comprend 1 à 16 atomes de carbone, et le radical alkyle linéaire ou ramifié comprend 1 à 16 atomes de carbone,
o un radical acyle dans lequel le radical alkyle linéaire, ou ramifié comprend 1 à 16 atomes de carbone,
o un radical aryle, de préférence phényle, éventuellement substitué par un ou plusieurs radicaux choisis parmi les radicaux amino, sulfo, alcoxy,
o un radical arylalkyle, de préférence benzyle, éventuellement substitué par un ou plusieurs radicaux choisis parmi les radicaux amino, sulfo, alcoxy,
o un hétérocycle avec un atome d'azote, à 5 à 12 chaînons, de préférence 5 à 7 chaînons, saturé ou non, comprenant éventuellement au moins un autre hétéroatome choisi parmi l'oxygène, l'azote ou le soufre, ledit hétérocycle étant éventuellement substitué par un ou plusieurs groupement(s) identiques ou non choisis parmi les radicaux alkyle ou hydroxyalkyle en C₁-C₁₆, linéaires ou ramifiés, les groupements carboxy, les groupements alcoxy carbonyle linéaires ou ramifiés dont l'alcoxy est en C₁-C₁₆, les groupements aminoalkylcarbamoyle avec le radical alkyle en C₁-C₁₆, linéaire ou ramifié ;
R₂ représente un groupement choisi parmi l'atome d'hydrogène, les atomes d'halogènes tels que F, Cl, Br et I, les radicaux alcoxy, les radicaux alkyle, le radical sulfonique, le radical carboxy ;
R₃, R₄, R₅, R₆, R₇, R₉ identiques ou non représentent chacun indépendamment un groupement choisi parmi l'atome d'hydrogène ; les atomes d'halogène tels que F, Cl, Br et I ; les radicaux alcoxy ; les radicaux alkyle ; les groupes amino ; les groupes amino, mono ou disubstitués par un ou plusieurs groupe(s), identiques ou non, choisi(s) parmi le groupe benzyle, les groupes aryle, et les groupes alkyle tels que les groupes monoaryl amino, diaryl amino, monoalkylamino, (dialkyl)amino dans lesquels les substituants du groupe amino tels que les radicaux alkyle, peuvent former ensemble avec l'atome d'azote auquel ils sont rattachés un hétérocycle de préférence saturé de 5 à 12 chaînons, de préférence de 5 à 7 chaînons comprenant éventuellement en outre au moins un autre hétéroatome choisi parmi les atomes d'azote, d'oxygène et de soufre, ledit hétérocycle pouvant être éventuellement substitué ; ou bien un ou les deux substituants du groupe amino tels que les radicaux alkyle peuvent former ensemble avec un substituant situé en position ortho du groupe amino mono ou disubstitué tel que monoalkylamino ou (dialkyl)amino un hétérocycle à 5 à 6 chaînons pouvant éventuellement comprendre en outre un ou plusieurs autres hétéroatomes choisis parmi les atomes d'azote, d'oxygène et de soufre, ledit hétérocycle pouvant être éventuellement substitué ;
les radicaux sulfoalkyle linéaire ou ramifié en C₁-C₁₆, de préférence en C₁-C₁₀ ;
les radicaux hydroxyalkyle linéaire ou ramifiée en C₁-C₁₆, de préférence en C₁-C₁₀ ;
les groupes mono-, di- arylamino dans lesquels la partie aryle est éventuellement substituée par un ou plusieurs substituants identiques ou différents, choisis parmi les atomes d'halogène ; les radicaux alkyle linéaire ou ramifié en C₁ à C₁₆, de préférence en C₁ à C₁₀ ; les radicaux alcoxy linéaire ou ramifié en C₁ à C₁₆, de préférence en C₁-C₁₀ ; les radicaux aryloxy éventuellement substitué ; mésyle (CH₃-SO₂-) ; amino ; amino mono ou disubstitué par un ou plusieurs groupements identiques ou non choisis parmi les radicaux alkyle linéaires ou ramifiés en C₁-C₄, phényle, dans lequel le ou les substituants du groupe amino tel que les groupements alkyle peuvent former, conjointement avec l'atome d'azote dudit groupement amino, auquel ils sont rattachés, un hétérocycle de préférence saturé, comprenant de 5 à 12 chaînons, de préférence de 5 à 7 chaînons, comprenant éventuellement un autre hétéroatome, choisi parmi l'oxygène, l'azote et le soufre ; ledit hétérocycle pouvant éventuellement être substitué, ledit hétérocycle pouvant éventuellement être condensé avec un cycle aromatique de préférence à 6 chaînons, ce dernier étant éventuellement substitué ;
L₁ est une chaîne alkyle, linéaire ou ramifiée en C₁-C₁₆ éventuellement interrompue par au moins un hétéroatome choisi parmi l'azote, l'oxygène et le soufre et/ou par au moins un groupement comprenant au moins un hétéroatome choisi parmi l'azote, l'oxygène et le soufre ;
An représente un anion ou un mélange d'anions, organiques ou minéraux par exemple choisi parmi un halogénure tel que chlorure, bromure, fluorure, iodure ; un hydroxyde ; un sulfate ; un hydrogénosulfate ; un alkylsulfate pour lequel la partie alkyle, linéaire ou ramifiée, est en C₁-C₆, comme l'ion méthylsulfate, éthylsulfate ; les carbonates et hydrogénocarbonates ; des sels d'acides carboxyliques tels que le formiate, l'acétate, le citrate, le tartrate, l'oxalate ; les alkylsulfonates pour lesquels la partie alkyle, linéaire ou ramifiée, est en C₁-C₆ comme l'ion méthylsulfonate ; les arylsulfonates pour lesquels la partie aryle, de préférence phényle, est éventuellement substituée par un ou plusieurs radicaux alkyle en C₁-C₄ tel que par exemple le 4-toluylsulfonate ; les alkylsulfonyles tel que le mésylate.

3. Composition de teinture selon la revendication 1 ou 2, **caractérisée en ce qu'**elle comprend un composé de formule (I), choisi parmi les composés suivants :
Sel interne de Xanthylium, 9-[4-[[(3-amino-4-sulfophenyl)amino]sulfonyl]-2-sulfophenyl]- 3,6-bis(dimethylamino
Sel interne de Xanthylium, 3,6-bis(diethylamino)-9-[2-sulfo-4-[[(4-sulfophenyl)amino]sulfonyl]phenyl]
Sel interne de Xanthylium, 3,6-bis(diethylamino)-9-[4-[[[2-(ethylthio)ethyl]amino]sulfonyl]-2-sulfophenyl]
Sel interne de Xanthylium, 3,6-bis(diethylamino)-9-[4-[[(4-methoxy-2-sulfophenyl)amino]sulfonyl]-2-sulfophenyl]
Sel interne de Xanthylium, 3,6-bis(diethylamino)-9-[4-[[(2-methoxy-4-sulfophenyl)amino]sulfonyl]-2-sulfophenyl]
Sel interne de Xanthylium, 9-[4-[[(3-amino-2-sulfophenyl)amino]sulfonyl]-2-sulfophenyl]- 3,6-bis(diethylamino)
Sel interne de Xanthylium, 9-[4-[[(3-amino-4-sulfophenyl)amino]sulfonyl]-2-sulfophenyl]- 3,6-bis(diethylamino)
Chlorure de Xanthylium, 9-[2-[(4-carboxy-1-piperidinyl)sulfonyl]phenyl]-3,6-bis(phenylamino)
Chlorure de Xanthylium, 3,6-bis(2,3-dihydro-1H-indol-1-yl)-9-[2-[[4-(ethoxycarbonyl)-1-piperidinyl]sulfonyl]phenyl]
Sel interne de Xanthylium, 9,9'-[dithiobis[2,1-ethanediyliminosulfonyl(2-sulfo-4,1-phenylene)]]bis[3,6-bis(diethylamino)
Chlorure de Xanthylium, 9-[2-[(dihexylamino)sulfonyl]phenyl]-3,6-bis(2,3-dihydro-2,3,3-trimethyl-1H-indol-1-yl)
Chlorure de Xanthylium, 3,6-bis[(2-chlorophenyl)methylamino]-9-[2-[(dihexylamino)sulfonyl]phenyl]
Sel interne de Xanthylium, 9-[4-[[[2-(carboxyamino)ethyl]amino]sulfonyl]-2-sulfophenyl]-3,6-bis(diethylamino)
Sel interne de Xanthylium, 9-[2-[[[2-(carboxyamino)ethyl]amino]sulfonyl]-4-sulfophenyl]-3,6-bis(diethylamino)
Sel interne de Xanthylium, 9-[4-[[(6-aminohexyl)amino]sulfonyl]-2-sulfophenyl]-3,6-bis(diethylamino)
Chlorure de Xanthylium, 9-[2-[[4-[[(5-aminopentyl)amino]carbonyl]-1-piperidinyl]sulfonyl]phenyl]-3,6-bis(methylphenylamino)
Sel interne de Xanthylium, 9-[2-[(4-carboxy-1-piperidinyl)sulfonyl]phenyl]-3,6-bis[methyl(4-sulfophenyl)amino]
Chlorure de Xanthylium, 9-[2-[(4-carboxy-1-piperidinyl)sulfonyl]phenyl]-3,6-bis(methylphenylamino)
Chlorure de Xanthylium, 9-[2-[[3-(hydroxymethyl)-1-piperidinyl]sulfonyl]phenyl]-3,6-bis(methylphenylamino) Xanthylium, 9-[2-[(4-carboxy-1-piperidinyl)sulfonyl]phenyl]-3,6-bis(methylphenylamino)
Sel interne de Xanthylium, 9-[4-[[[3-[2-[2-(3-aminopropoxy)ethoxy]ethoxy]propyl]amino]sulfonyl]-2-sulfophenyl]-3,6-bis(diethylamino)
Sel interne de Xanthylium, 9-[4-[[(2-aminoethyl)amino]sulfonyl]-2-sulfophenyl]-3,6-bis(diethylamino)
Sel interne de Xanthylium, 9-[2-[[(5-carboxypentyl)amino]sulfonyl]-4-sulfophenyl]-3,6-bis(diethylamino)
Sel interne de Xanthylium, 3,6-bis(diethylamino)-9-[2-[[(6-methoxy-6-oxohexyl)amino]sulfonyl]-4-sulfophenyl
Sel interne de Xanthylium, 3,6-bis(diethylamino)-9-[4-[[(6-methoxy-6-oxohexyl)amino]sulfonyl]-2-sulfophenyl]
Sel interne dihydrate de Xanthylium, 3,6-bis(diethylamino)-9-[4-[(methylamino)sulfonyl]-2-sulfophenyl]
Chlorure de, Xanthylium, 3,6-bis(diethylamino)-9-[2-[(methylamino)sulfonyl]-4-sulfophenyl]-,
Chlorure de Xanthylium, 9-[2-(aminosulfonyl)phenyl]-3-[(2-sulfophenyl)amino]- mono sel de sodium
Sel interne de Xanthylium, 3,6-bis(diethylamino)-9-[4-(1-piperazinylsulfonyl)-2-sulfophenyl]
Bromure de Xanthylium, 3,6-bis[[2-(1,1-dimethylethyl)phenyl]amino]-9-[2-[(ethylamino)sulfonyl]phenyl]
Hexafluorophosphate de Xanthylium, 9-[2-[(acetylmethylamino)sulfonyl]phenyl]-3,6-bis(methylphenylamino)
Xanthylium, 9-[2-[(acetylmethylamino)sulfonyl]phenyl]-3,6-bis(methylphenylamino)-Chlorure de Xanthylium, 3-[(2-bromo-5-sulfophenyl)amino]-9-[2-[[(2-hydroxyethyl)amino]sulfonyl]phenyl]-6-[(1,1,3,3-tetramethylbutyl)amino] mono sel de sodium
Chlorure de Xanthylium, 3-[(2-fluoro-5-sulfophenyl)amino]-9-[2-[[(2-hydroxyethyl)amino]sulfonyl]phenyl]-6-[(1,1,3,3-tetramethylbutyl)amino] mono sel de sodium
Chlorure de Xanthylium, 3-[(2,6-dimethylphenyl)amino]-9-[2-[[(2-hydroxyethyl)amino]sulfonyl]phenyl]-6-[(2-methoxy-5-sulfophenyl)amino] mono sel de sodium Sel interne de Xanthylium, 9-[4-[[(5-carboxypentyl)amino]sulfonyl]-2-sulfophenyl]-3,6-bis(diethylamino)
Sel interne de Xanthylium, 9-[4-[(cyclohexylamino)sulfonyl]-2-sulfophenyl]-3,6-bis(diethylamino)
Sel interne de Xanthylium, 3,6-bis(diethylamino)-9-[4-[[(3-ethoxypropyl)amino]sulfonyl]-2-sulfophenyl]
Sel interne de Xanthylium, 3,6-bis(diethylamino)-9-[4-[[[3-[(2-ethylhexyl)oxy]propyl]amino]sulfonyl]-2-sulfophenyl]
Sel interne de Xanthylium, 9-[4-[[(3-carboxypropyl)amino]sulfonyl]-2-sulfophenyl]-3,6-bis(diethylamino)
Xanthylium, 9-[2-[(acetylmethylamino)sulfonyl]phenyl]-3,6-bis[2,3-dihydro-5-[[[3-(trimethylammonio)propyl]amino]sulfonyl]-1H-indol-1-yl]-Di iodure de 1-Propanaminium, 3,3'-[[9-[2-[(acetylmethylamino)sulfonyl]phenyl]-9H-xanthene-3,6-diyl]bis[(2,3-dihydro-1H-indole-1,5-diyl)sulfonylimino]]bis[N,N,N-trimethyl
Sel interne de Xanthylium, 9-[4-[[[2-[[(2-aminoethoxy)methoxy]methoxy]ethyl]amino]sulfonyl]-2-sulfophenyl]-3,6-bis(diethylamino)
Sel interne de Xanthylium, 3,6-bis(diethylamino)-9-[2-[(methylamino)sulfonyl]-4-sulfophenyl] Sel interne de Xanthylium, 3,6-bis(diethylamino)-9-[4-[(methylamino)sulfonyl]-2-sulfophenyl]
Sel interne de Xanthylium, 9-[4-[[(11-carboxyundecyl)amino]sulfonyl]-2-sulfophenyl]-3,6-bis(diethylamino)
4-methylbenzenesulfonate de Xanthylium, 9-[2-(aminosulfonyl)phenyl]-3,6-bis(diethylamino)
Sel interne de [9-[4-[Bis(2-hydroxyethyl)sulfamoyl]-2-sulfophenyl]-6-(diethylamino)-3H-xanthen-3-ylidene]diethylammonium hydroxide
Sel interne de [9-[4-[(2-Chloroethyl)sulfamoyl]-2-sulfophenyl]-6-(diethylamino)-3H-xanthen-3-ylidene]diethylammonium hydroxide
Sel interne de [9-[4-[Bis(2-chloroethyl)sulfamoyl]-2-sulfophenyl]-6-(diethylamino)-3H-xanthen-3-ylidene]diethylammonium hydroxide
Sel interne de [6-(Diethylamino)-9-[4-(phenylsulfamoyl)-2-sulfophenyl]-3H-xanthen-3-ylidene]diethylammonium hydroxide
Sel interne de Xanthylium, 3,6-bis(diethylamino)-9-[4-[[(2-hydroxyethyl)amino]sulfonyl]-2-sulfophenyl]
Sel interne de [6-(Diethylamino)-9-(4-sulfamoyl-2-sulfophenyl)-3H-xanthen-3-ylidene]diethylammonium hydroxide
Sel interne de Xanthylium, 3,6-bis[5-[(dodecylamino)sulfonyl]-2,3-dihydro-1H-indol-1-yl]-9-(2-sulfophenyl)
Xanthylium, 3,6-bis(2,3-dihydro-1H-indol-1-yl)-9-[2-[(methylamino)sulfonyl]phenyl]
Xanthylium, 9-[2-[(methylamino)sulfonyl]phenyl]-3-[methyl[2- (methylsulfonyl)phenyl]amino]-6-[methyl[4-(methylsulfonyl)phenyl]amino]
Sel interne de Xanthylium, 3-[[4-[(dimethylamino)sulfonyl]phenyl]methylamino]-6-(methylphenylamino)-9-(2-sulfophenyl) chlorure de Xanthylium, 9-[2-(aminosulfonyl)phenyl]-3,6-bis(diethylamino)
Sel interne de Xanthylium, 3,6-bis[[5-carboxy-2-[(methylsulfonyl)amino]phenyl]amino]-9-(2-carboxyphenyl)
Sel interne de Xanthylium, 9-(2-carboxyphenyl)-3,6-bis[ethyl[2- [(ethylsulfonyl)amino]phenyl]amino]
Sel interne de Xanthylium, 3-[ethyl[2-methyl-5-[(methylsulfonyl)amino]phenyl]amino]-6-[[2-methyl-5-[(methylsulfonyl)amino]phenyl](3-sulfopropyl)amino]-9-(2-sulfophenyl), mono sel de sodium
Sel interne de Xanthylium, 3,6-bis[methyl[2-[(methylsulfonyl)amino]phenyl]amino]-9-(2-sulfophenyl)
Sel interne de Xanthylium, 3,6-bis[ethyl[2-methyl-5-[(methylsulfonyl)amino]phenyl]amino]- 9-(2-sulfophenyl)
Sel interne de Xanthylium, 3-[ethyl(2-methylphenyl)amino]-6-[[2-methyl-5-[(methylsulfonyl)amino]phenyl]amino]-9-(2-sulfophenyl)
Sel interne de Xanthylium, 3-[(2,6-dimethylphenyl)amino]-9-(2,4-disulfophenyl)-6-[[2-[(methylsulfonyl)amino]phenyl]amino] mono sel de sodium Sel interne de Xanthylium, 3-amino-6-[[2-chloro-4-[2-[(methylsulfonyl)amino]phenoxy]phenyl]amino]-9-(2-sulfophenyl)
Sel interne de Xanthylium, 3-amino-6-[[2-fluoro-4-[2-[(methylsulfonyl)amino]phenoxy]phenyl]amino]-9-(2-sulfophenyl)
Sel interne de Xanthylium, 3-[(4-cyano-2-methoxyphenyl)amino]-6-[[4- [(methylsulfonyl)amino]-2-propoxyphenyl]amino]-9-(2-sulfophenyl)
Sel interne de Xanthylium, 3-amino-6-[[2-methoxy-5-[2-[(methylsulfonyl)amino]phenoxy]phenyl]amino]-9-(2-sulfophenyl)
Sel interne de Xanthylium, 3-[[5-(aminosulfonyl)-2-methoxyphenyl]amino]-6-[[4- [(ethoxycarbonyl)amino]-2-methoxyphenyl]amino]-9-(2-sulfophenyl)
Sel interne de Xanthylium, 3,6-bis[ethyl[2-[(methylsulfonyl)amino]ethyl]amino]-9-(2,4,5-tricarboxyphenyl)
Sel interne de Xanthylium, 9-(2,4-disulfophenyl)-3,6-bis[ethyl[2- [(methylsulfonyl)amino]ethyl]amino] mono sel de lithium
Sel interne de Xanthylium, 3,6-bis[ethyl[2-[(methylsulfonyl)amino]ethyl]amino]-9-(2-sulfophenyl)
Sel interne de Xanthylium, 3-[[[[(5-carboxypentyl)amino]sulfonyl]-2-methylphenyl]amino]-9-(2-carboxyphenyl)-6-[(2-methylphenyl)amino]
Sel interne de Xanthylium, 9-(2-carboxyphenyl)-3-[[4-[[(3-carboxypropyl)amino]sulfonyl]- 2,6-dimethylphenyl]amino]-6-[(2,6-dimethylphenyl)amino]
Sel interne de Xanthylium, 3,6-bis[[4-[[(2-carboxyphenyl)amino]sulfonyl]-2,6-dimethylphenyl]amino]-9-(2-sulfophenyl)
Sel interne de Xanthylium, 3,6-bis[5-[(dodecylamino)sulfonyl]-2,3-dihydro-1H-indol-1-yl]-9-(2-sulfophenyl)
Sel interne de Xanthylium, 3-[[4-[(dimethylamino)sulfonyl]phenyl]methylamino]-6-(methylphenylamino)-9-(2-sulfophenyl)
Sel interne de Xanthylium, 3,6-bis[[4-[(dimethylamino)sulfonyl]phenyl]amino]-9-(2-sulfophenyl)
Sel interne de Xanthylium, 3-chloro-6-[[4-[(dimethylamino)sulfonyl]phenyl]amino]-9-(2-sulfophenyl)
Sel interne de Xanthylium, 3,6-bis[[4-[(dimethylamino)sulfonyl]phenyl]methylamino]-9-(2-sulfophenyl)
Sel interne de Xanthylium, 3-[[4-[(dimethylamino)sulfonyl]phenyl]amino]-6-(phenylamino)-9- (2-sulfophenyl)
Sel interne de Xanthylium, 3,6-bis[[4-[(dimethylamino)sulfonyl]-2-methoxyphenyl]amino]-9-(2-sulfophenyl)
Sel interne de Xanthylium, 3,6-bis[[4-(aminosulfonyl)phenyl]amino]-9-(2-sulfophenyl
Sel interne de 9-{4-[Bis-(2-hydroxy-ethyl)-sulfamoyl]-2-sulfo-phenyl}-3,6-bis-diethylamino-xanthenylium
Sel interne de 3,6-Bis-diethylamino-9-(4-dipropylsulfamoyl-2-sulfo-phenyl)-xanthenylium
Sel interne de 3,6-Bis-diethylamino-9-[4-(4-methylpiperazine-1-sulfonyl)-2-sulfo-phenyl]- xanthenylium
Sel interne de 3,6-Bis-diethylamino-9-[4-(3-imidazol-1-yl-propylsulfamoyl)-2-sulfo-phenyl] -xanthenylium
Sel interne de 9-{2-[Bis-(2-hydroxy-ethyl)-sulfamoyl]-4-sulfo-phenyl}-3,6-bis-diethylamino- xanthenylium
Sel interne de 3,6-Bis-diethylamino-9-[2-(4-methylpiperazine-1-sulfonyl)-4-sulfo-phenyl]- - xanthenylium.

4. Composition selon l'une quelconque des revendications précédentes, **caractérisée en ce que** le ou les composé(s) de formule (I) et/ou le ou les sel(s) d'addition représentent de 0,0001 à 10% en poids du poids total de la composition de teinture, de préférence de 0,005 à 10% en poids et, de préférence encore, de 0,01 à 6% en poids du poids total de la composition de teinture.

5. Composition selon l'une quelconque des revendications précédentes, **caractérisée en ce qu'**elle contient, en outre, un ou plusieurs colorant(s) direct(s) différent(s) des composés de formules (I).

6. Composition selon la revendication 5, **caractérisée en ce que** le ou les colorant(s) direct(s) différent(s) des composés de formule (I) sont choisis parmi les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques les colorants directs naturels et les colorants directs xanthéniques différents des composés de formule (I).

7. Composition selon l'une quelconque des revendications 5 et 6, **caractérisée en ce que** le ou les colorant(s) direct(s) différent(s) des composés de formules (I) représentent de 0,001 à 20% en poids, de préférence de 0,005 à 10% en poids du poids total de la composition de teinture.

8. Composition selon l'une quelconque des revendications précédentes, **caractérisée en ce qu'**elle contient, en outre, une ou plusieurs base(s) d'oxydation et éventuellement un ou plusieurs coupleur(s).

9. Composition selon la revendication 8, **caractérisé en ce que** la ou les base (s) d'oxydation sont choisies parmi les paraphénylènediamines, les bisphénylalkylènediamines, les para-aminophénols, les ortho-aminophénols, les bases hétérocycliques et leurs sels d'addition.

10. Composition selon la revendication 8, **caractérisée en ce que** le ou les coupleur(s) sont choisi(s) parmi les coupleurs métaphénylènediamines, les coupleurs méta-aminophénols, les coupleurs métadiphénols, les coupleurs naphtaléniques, les coupleurs hétérocycliques et leurs sels d'addition.

11. Composition selon l'une quelconque des revendications 8 à 10, **caractérisée en ce que** le ou les base(s) d'oxydation et/ou le ou les coupleur(s) sont présents chacun en une quantité de 0,001 à 10% en poids, de préférence de 0,005 à 6% en poids du poids total de la composition de teinture.

12. Composition selon l'une quelconque des revendications précédentes, **caractérisée en ce qu'**elle a un pH de 3 à 12, de préférence de 5 à 11, de préférence encore de 6 à 8,5.

13. Procédé de teinture directe des fibres kératiniques et en particulier des fibres kératiniques humaines, telles que les cheveux, comprenant l'application d'au moins une composition de teinture selon l'une quelconque des revendications 1 à 12 contenant au moins un composé de formule (I) sur les fibres kératiniques puis l'observation d'un temps de pause suivi du rinçage des fibres.

14. Procédé selon la revendication 13, **caractérisé en ce que** le temps de pause est de 3 à 50 minutes, de préférence de 5 à 30 minutes.

15. Procédé de teinture des fibres kératiniques et, en particulier, des fibres kératiniques humaines, telles que les cheveux, dans lequel on applique sur lesdites fibres au moins une composition de teinture selon l'une quelconques des revendications 1 à 12 comprenant au moins un composé de formule (I) et éventuellement au moins une base d'oxydation et éventuellement un ou plusieurs coupleur(s), la couleur étant révélée à pH acide, neutre ou alcalin, à l'aide d'un agent oxydant.

16. Procédé selon la revendication 15, **caractérisé en ce que** l'agent oxydant est choisi parmi le peroxyde d'hydrogène, le peroxyde d'urée, les bromates de métaux alcalins, les persels tels que les perborates et persulfates, les peracides et les enzymes oxydases, tel que les peroxydases, les oxydo-réductases à 2 électrons, comme les uricases et les oxygenases à 4 électrons, comme les laccases.

17. Dispositif à plusieurs compartiments comprenant un premier compartiment renfermant une composition tinctoriale selon l'une quelconque des revendications 1 à 12, comprenant au moins un composé de formule (I) et éventuellement au moins une base d'oxydation et éventuellement un ou plusieurs coupleurs, et un deuxième compartiment renfermant une composition oxydante.

18. Dispositif à plusieurs compartiments comprenant un premier compartiment qui renferme une composition tinctoriale selon l'une quelconque des revendications 1 à 12, comprenant au moins un composé de formule (I), un second compartiment renferme une composition tinctoriale comprenant au moins une base d'oxydation et éventuellement au moins un coupleur, et un troisième compartiment renferme une composition oxydante.

## Claims

1. Composition for dyeing keratin fibres comprising in a cosmetic medium suitable for dyeing, at least one dye selected from the compounds complying with formula (I) below, the mesomeric forms thereof, and the oligomers thereof: 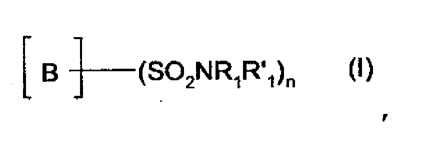 wherein B represents: 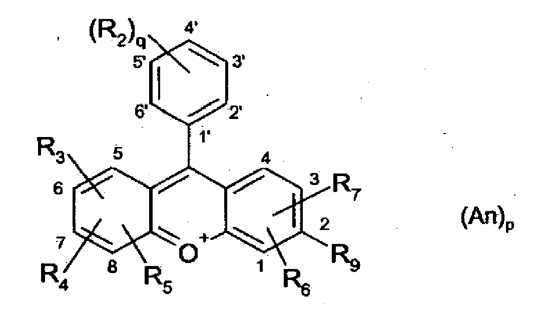 wherein R₁, R₂, identical or not, represent:
- a hydrogen atom,
- an alkyl radical,
- a thioalkyl radical,
- an alcoxy radical,
- an amino alkyl radical,
- a trialkylammonioalkyl radical,
- a carboxy alkyl radical,
- a carboxy amino alkyl radical,
- an amino alcoxy polyalcoxy radical wherein the alcoxy radicals of the polyalcoxy group, identical or not, each comprise 1 to 4 carbon atoms,
- an alcoxycarbonyl radical,
- an alcoxycarbonylalkyl radical,
- an alcoxyalkyl radical,
- an acyl radical,
- an aryl, preferably phenyl, radical,
- an arylalkyl, preferably benzyl, radical,
- a heterocycle with a nitrogen atom, having 5 to 12 members, preferably 5 to 7 members, saturated or not, optionally comprising at least one other heteroatom selected from oxygen, nitrogen or sulphur, said heterocycle being optionally substituted;
R'₁ also possibly representing the following group: 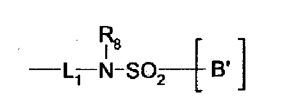
wherein:
- R₈ represents a group selected from those defining R₁ and R'₁,
- L₁ represents a divalent hydrocarbon chain comprising 1 to 16 carbon atoms, optionally interrupted with at least one heteroatom selected from oxygen, sulphur or nitrogen, and/or by a group comprising at least one heteroatom, such as for example CO, SO₂,
- B' represents a group according to formula (B) and is identical or not to B;
the radicals R₁ and R'₁ optionally form together with the nitrogen atom with which they are attached, a heterocycle, preferably saturated, comprising 5 to 12 members, preferably 5 to 7 members, optionally comprising another heteroatom selected from oxygen, nitrogen or sulphur, said heterocycle being optionally substituted;
- R₂ represents a group selected from the hydrogen atom, halogen atoms such as F, Cl, Br and I, alcoxy radicals, alkyl radicals, the sulphonic radical, the carboxy radical;
- R₃, R₄, R₅, R₆, R₇, R₉ each represent independently a group selected from the hydrogen atom; halogen atoms such as F, Cl, Br and I; alcoxy radicals; alkyl radicals; amino groups; amino groups, mono or disubstituted by a group selected from the benzyl group, the aryl groups, and the alkyl groups, such as monoaryl amino, diaryl amino, monoalkylamino, (dialkyl)amino groups wherein the amino group substituents such as alkyl radicals, may form together with the nitrogen atom with which they are bound a heterocycle, preferably saturated having 5 to 12 members, preferably 5 to 7 members, optionally comprising at least one other heteroatom selected from oxygen, nitrogen or sulphur, said heterocycles being optionally substituted; or one or both amino group substituents such as alkyl radicals may form together with a substituent located in the ortho position of the mono or disubstituted amino group such as monoalkylamino or (dialkyl)amino, a heterocycle having 5 to 6 members optionally also comprising at least one other heteroatom selected from nitrogen, oxygen and sulphur atoms, said heterocycle possibly being optionally substituted;
the group (s) -SO₂NR₁R'₁ and/or -L₁NR₈SO₂- being bound either directly onto one or more cycles bearing substituents R₂ to R₉ or via one of more of the substituents R₂ to R₉;
the coefficient n is an integer ranging from 1 to 4, preferably equal to 1;
the coefficient q is an integer ranging from 1 to 3, preferably equal to 1;
An represents a mono or polyvalent anion, or a mixture of anions;
the coefficient p is equal to 0 or 1;
An and p being such that the compound is electrically neutral.

2. Dyeing composition according to claim 1, wherein, in the formula (I), R₁, R'₁, identical or not, represent:
- a hydrogen atom,
- a linear or branched C₁-C₁₆, or cyclic C₃-C₁₆, alkyl radical, optionally substituted by one or more groups, identical or not, selected from the amino, sulphonic, and alcoxy groups,
- a linear, branched C₁-C₁₆, or cyclic C₃-C₁₆ hydroxyalkyl radical,
- a linear, branched C₁-C₁₆, or cyclic C₃-C₁₆ thioalkyl radical,
- a linear or branched C₁-C₁₆ alcoxy radical,
- a linear or branched C₁-C₁₆, or cyclic C₃-C₁₆ amino alkyl radical,
- a trialkylammonioalkyl radical wherein the alkyl radicals, identical or not, are in C₁-C₁₆,
- a linear or branched halogenoalkyl radical wherein the linear or branched alkyl radical comprises 1 to 16 carbon atoms,
- a linear, branched C₁-C₁₆, or cyclic C₃-C₁₆ carboxy alkyl radical,
- a linear or branched C₁-C₁₆, or cyclic C₃-C₁₆ carboxy amino alkyl,
- a polyalcoxy alcoxy amino radical wherein the linear or branched alcoxy radicals, identical or not, comprise 1 to 4 carbon atoms,
- an alcoxycarbonyl radical wherein the linear or branched alcoxy radical comprises 1 to 16 carbon atoms,
- an alcoxycarbonylalkyl radical wherein the linear or branched alcoxy radical comprises 1 to 16 carbon atoms and the linear or branched alkyl radical comprises 1 to 16 carbon atoms,
- an alcoxyalkyl radical wherein the linear or branched alcoxy radical comprises 1 to 16 carbon atoms, and the linear or branched alkyl radical comprises 1 to 16 carbon atoms,
- an acyl radical wherein the linear or branched alkyl radical comprises 1 to 16 carbon atoms,
- an aryl, preferably phenyl, radical substituted by one or more radicals selected from the amino, sulpho, alcoxy radicals,
- an arylalkyl, preferably benzyl, radical optionally substituted by one or more radicals selected from the amino, sulpho, alcoxy radicals,
- a heterocycle with a nitrogen atom, having 5 to 12 members, preferably 5 to 7 members, saturated or not, optionally comprising at least one other heteroatom selected from oxygen, nitrogen or sulphur, said heterocycle being optionally substituted by one or group(s), identical or not, selected from linear or branched C₁-C₁₆ alkyl or hydroxyalkyl radicals, carboxy groups, linear or branched carbonyl alcoxy groups wherein the alcoxy is in C₁-C₁₆, aminoalkylcarbamoyl groups with the linear or branched C₁-C₁₆ alkyl radical;
R₂ represents a group selected from the hydrogen atom, halogen atoms such as F, Cl, Br and I, alcoxy radicals, alkyl radicals, the sulphonic radical, the carboxy radical;
R₃, R₄, R₅, R₆, R₇, R₉, identical or not, each represent independently a group selected from the hydrogen atom; halogen atoms such as F, Cl, Br and I; alcoxy radicals; alkyl radicals; amino groups; amino groups, mono or disubstituted by one or more group(s), identical or not, selected from the benzyl group, the aryl groups, and the alkyl groups, such as monoaryl amino, diaryl amino, monoalkylamino, (dialkyl)amino groups wherein the amino group substituents such as alkyl radicals may form together with the nitrogen atom with which they are bound a heterocycle, preferably saturated having 5 to 12 members, preferably 5 to 7 members, optionally comprising at least one other heteroatom selected from nitrogen, oxygen or sulphur atoms, said heterocycle being optionally substituted; or one or both amino group substituents such as alkyl radicals may form together with a substituent located in the ortho position of the mono or disubstituted amino group such as monoalkylamino or (dialkyl)amino a heterocycle having 5 to 6 members optionally also comprising at least one other heteroatom selected from nitrogen, oxygen and sulphur atoms, said heterocycle possibly being optionally substituted;
the linear or branched C₁-C₁₆, preferably C₁-C₁₀, sulphoalkyl radicals;
the linear or branched C₁-C₁₆, preferably C₁-C₁₀, hydroxyalkyl radicals;
the mono-, di- arylamino groups wherein the aryl part is optionally substituted by one or more identical or different substituents, selected from halogen atoms; linear or branched C₁-C₁₆, preferably C₁-C₁₀ alkyl radicals; linear or branched C₁-C₁₆, preferably C₁-C₁₀ alcoxy radicals; optionally substituted aryloxy radicals; mesyl (CH₃-SO₂-); amino; amino mono or disubstituted by one or more groups, identical or not, selected from linear or branched C₁-C₄ alkyl radicals, phenyl radicals, wherein the amino group substituent(s) such as the alkyl groups may form, in conjunction with the nitrogen atom of said amino group, with which they are bound, a preferably saturated heterocycle, comprising 5 to 12 members, preferably 5 to 7 members, optionally comprising another heteroatom, selected from oxygen, nitrogen and sulphur; said heterocycle optionally being substituted, said heterocycle optionally being condensed with an aromatic cycle preferably having 6 members, the latter being optionally substituted;
L₁ is a linear or branched C₁-C₁₆ alkyl chain, optionally interrupted with at least one heteroatom selected from oxygen, sulphur and nitrogen and/or by at least one group comprising at least one heteroatom selected from nitrogen, oxygen and sulphur;
An represents an anion, or a mixture of organic or mineral anions for example selected from a halide such as chloride, bromide, fluoride, iodide; a hydroxide; a sulphate; a hydrogen sulphate; an alkylsulphate for which the linear or branched alkyl part is in C₁-C₆, such as the methylsulphate, ethylsulphate ion; carbonates and hydrogen carbonates; carboxylic acid salts such as formiate, acetate, citrate, tartrate, oxalate; alkylsulphonates for which the linear or branched alkyl part is in C₁-C₆ such as the methylsulphonate ion; arylsulphonates for which the aryl, preferably phenyl, part is optionally substituted by one or more C₁-C₄ alkyl radicals such as for example 4-toluylsulphonate; alkylsulphonates such as mesylate.

3. Dyeing composition according to claim 1 or 2, **characterised in that** it comprises a compound according to formula (I), selected from the following compounds:
Internal Xanthylium salt, 9-[4-[[(3-amino-4-sulphophenyl)amino]sulphonyl]-2-sulphophenyl]-3,6-bis(dimethylamino)
Internal Xanthylium salt, 3,6-bis(diethylamino)-9-[2-sulpho-4-[[(4-sulphophenyl)amino]sulphonyl]phenyl-]
Internal Xanthylium salt, 3,6-bis(diethylamino)-9-[4-[[[2-(ethylthio)ethyl]amino]sulphonyl]-2-sulphophenyl]
Internal Xanthylium salt, 3,6-bis(diethylamino)-9-[4-[[(4-methoxy-2-sulphophenyl)amino]sulphonyl]-2-sulphophenyl]
Internal Xanthylium salt, 3,6-bis(diethylamino)-9-[4-[[(2-methoxy-4-sulphophenyl)amino]sulphonyl]-2-sulphophenyl]
Internal Xanthylium salt, 9-[4-[[(3-amino-2-sulphophenyl)-amino]sulphonyl]-2-sulphophenyl]-3,6-bis(diethylamino)
Internal Xanthylium salt, 9-[4-[[(3-amino-4-sulphophenyl)-amino]sulphonyl]-2-sulphophenyl]-3,6-bis(diethylamino)
Xanthylium chloride, 9-[2-[(4-carboxy-1-piperidinyl)sulphonyl]phenyl]-3,6-bis(phenylamino)
Xanthylium chloride, 3,6-bis(2,3-dihydro-1H-indol-1-yl)-9-[2-[[4-(ethoxycarbonyl)-1-piperidiny-1]sulphonyl]phenyl]
Internal Xanthylium salt, 9,9'-[dithiobis[2,1-ethanediyliminosulphonyl(2-sulpho-4,1-phenylene)]]bis[3,6-bis(diethylamino)]
Xanthylium chloride, 9-[2-[(dihexylamino)sulphonyl]phenyl]-3,6-bis(2,3-dihydro-2,3,3-trimethyl-1H-indol-1-yl)
Xanthylium chloride, 3,6-bis[(2-chlorophenyl)methylamino]-9-[2-[(dihexylamino)sulphonyl]phenyl]
Internal Xanthylium salt, 9-[4-[[[2-(carboxyamino)ethyl]amino]sulphonyl]-2-sulphophenyl]-3,6-bis(diethylamino)
Internal Xanthylium salt, 9-[2-[[[2-(carboxyamino)ethyl]-amino]sulphonyl]-4-sulphophenyl]-3,6-bis(diethylamino)
Internal Xanthylium salt, 9-[4-[[(6-aminohexyl)amino]-sulphonyl]-2-sulphophenyl]-3,6-bis(diethylamino)
Xanthylium chloride, 9-[2-[[4-[[(5-aminopentyl)amino]carbonyl]-1-piperidinyl]sulphonyl]phenyl]-3,6-bis(methylphenylamino)
Internal Xanthylium salt, 9-[2-[(4-carboxy-1-piperidinyl)-sulphonyl]phenyl]-3,6-bis[methyl(4-sulphophenyl)amino]
Xanthylium chloride, 9-[2-[(4-carboxy-1-piperidinyl)sulphonyl]phenyl]-3,6-bis(methylphenylamino)
Xanthylium chloride, 9-[2-[[3-(hydroxymethyl)-1-piperidinyl]-sulphonyl]phenyl]-3,6-bis(methylphenylamino)
Xanthylium, 9-[2-[(4-carboxy-1-piperidinyl)sulphonyl]phenyl]-3,6-bis(methylphenylamino)
Internal Xanthylium salt, 9-[4-[[[3-[2-[2-(3-amino-propoxy)ethoxy]ethoxy]propyl]amino]sulphonyl]-2-sulphophenyl]-3,6-bis(diethylamino)
Internal Xanthylium salt, 9-[4-[[(2-aminoethyl)amino]sulphonyl]-2-sulphophenyl]-3,6-bis(diethylamino)
Internal Xanthylium salt, 9-[2-[[(5-carboxypentyl)amino]-sulphonyl]-4-sulphophenyl]-3,6-bis(diethylamino)
Internal Xanthylium salt, 3,6-bis(diethylamino)-9-[2-[[(6-methoxy-6-oxohexyl)amino]sulphonyl]-4-sulphophenyl]
Internal Xanthylium salt, 3,6-bis(diethylamino)-9-[4-[[(6-methoxy-6-oxohexyl)amino]sulphonyl]-2-sulphophenyl]
Internal dihydrate salt of Xanthylium, 3,6-bis(diethylamino)-9-[4-[(methylamino)sulphonyl]-2-sulphophenyl]
Xanthylium chloride, 3,6-bis(diethylamino)-9-[2-[(methylamino)-sulphonyl]-4-sulphophenyl]-,
Xanthylium chloride, 9-[2-(aminosulphonyl)phenyl]-3-[(2-sulphophenyl)amino]- monosodium salt
Internal Xanthylium salt, 3,6-bis(diethylamino)-9-[4-(1-piperazinylsulphonyl)-2-sulphophenyl]
Xanthylium bromide, 3,6-bis[[2-(1,1-dimethylethyl)phenyl]amino]-9-[2-[(ethylamino)sulphonyl]phenyl]
Xanthylium hexafluorophosphate, 9-[2-[(acetylmethylamino)sulphonyl]phenyl]-3,6-bis(methylphenylamino)
Xanthylium, 9-[2-[(acetylmethylamino)sulphonyl]phenyl]-3,6-bis(methylphenylamino)
Xanthylium chloride, 3-[(2-bromo-5-sulphophenyl)amino]-9-[2-[[(2-hydroxyethyl)amino]sulphonyl]phenyl]-6-[(1,1,3,3-tetramethylbutyl)amino], monosodium salt
Xanthylium chloride, 3-[(2-fluoro-5-sulphophenyl)amino]-9-[2-[[(2-hydroxyethyl)amino]sulphonyl]phenyl]-6-[(1,1,3,3-tetramethylbutyl)amino] monosodium salt
Xanthylium chloride, 3-[(2,6-dimethylphenyl)amino]-9-[2-[[(2-hydroxyethyl)amino]sulphonyl]phenyl]-6-[(2-methoxy-5-sulphophenyl)amino] monosodium salt
Internal Xanthylium salt, 9-[4-[[(5-carboxypentyl)-amino]sulphonyl]-2-sulphophenyl]-3,6-bis(diethylamino)
Internal Xanthylium salt, 9-[4-[(cyclohexylamino)sulphonyl]-2-sulphophenyl]-3,6-bis(diethylamino)
Internal Xanthylium salt, 3,6-bis(diethylamino)-9-[4-[[(3-ethoxypropyl)amino]sulphonyl]-2-sulphopheny- 1]
Internal Xanthylium salt, 3,6-bis(diethylamino)-9-[4-[[[3-[(2-ethylhexyl)oxy]propyl]amino]sulphonyl]-2-sulphophenyl]
Internal Xanthylium salt, 9-[4-[[(3-carboxypropyl)amino]-sulphonyl]-2-sulphophenyl]-3,6-bis(diethylamino)
Xanthylium, 9-[2-[(acetylmethylamino)sulphonyl]phenyl]-3,6-bis[2,3-dihydro-5-[[[3-(trimethylammonio)propyl]amino]sulphonyl]-1H-indol-1-yl]
1-Propanaminium diiodide, 3,3'-[[9-[2-[(acetylmethylamino)-sulphonyl]phenyl]-9H-xanthene-3,6-diyl]bi- s[(2,3-dihydro-1H-indole-1,5-diyl)sulphonylimino]]bis[N,N,N-trimethyl]
Internal Xanthylium salt, 9-[4-[[[2-[[(2-aminoethoxy)-methoxy]methoxy]ethyl]amino]sulphonyl]-2-sulpho- phenyl]-3,6-bis(diethylamino)
Internal Xanthylium salt, 3,6-bis(diethylamino)-9-[2-[(methylamino)sulphonyl]-4-sulphophenyl]
Internal Xanthylium salt, 3,6-bis(diethylamino)-9-[4-[(methylamino)sulphonyl]-2-sulphophenyl]
Internal Xanthylium salt, 9-[4-[[(11-carboxyundecyl)-amino]sulphonyl]-2-sulphophenyl]-3,6-bis(diethylamino)
Xanthylium 4-methylbenzenesulphonate, 9-[2-(aminosulphonyl)phenyl]-3,6-bis(diethylamino)
Internal salt of [9-[4-[bis(2-hydroxyethyl)sulphamoyl]-2-sulphophenyl]-6-(diethylamino)-3H-xanthen-3-ylidene]diethylammonium hydroxide
Internal salt of [9-[4-[(2-chloroethyl)sulphamoyl]-2-sulphophenyl]-6-(diethylamino)-3H-xanthen-3-ylidene]diethylammonium hydroxide
Internal salt of [9-[4-[bis(2-chloroethyl)sulphamoyl]-2-sulphophenyl]-6-(diethylamino)-3H-xanthen-3-ylidene]diethylammonium hydroxide
Internal salt of [6-(diethylamino)-9-[4-(phenylsulphamoyl)-2-sulphophenyl]-3H-xanthen-3-ylidene]diethylammonium hydroxide
Internal Xanthylium salt, 3,6-bis(diethylamino)-9-[4-[[(2-hydroxyethyl)amino]sulphonyl]-2-sulphopheny- 1]
Internal salt of [6-(diethylamino)-9-(4-sulphamoyl-2-sulphophenyl)-3H-xanthen-3-ylidene]diethylammonium hydroxide
Internal Xanthylium salt, 3,6-bis[5-[(dodecylamino)sulphonyl]-2,3-dihydro-1H-indol-1-yl]-9-(2-sulphophenyl)
Xanthylium, 3,6-bis(2,3-dihydro-1H-indol-1-yl)-9-[2-[(methylamino)sulphonyl]phenyl]
Xanthylium, 9-[2-[(methylamino)sulphonyl]phenyl]-3-[methyl[2-(methylsulphonyl)phenyl]amino]-6-[methyl[4-(methylsulphonyl)phenyl]amino]
Internal Xanthylium salt, 3-[[4-[(dimethylamino)sulphonyl]-phenyl]methylamino]-6-(methylphenylamino)- -9-(2-sulphophenyl)
Xanthylium chloride, 9-[2-(aminosulphonyl)phenyl]-3,6-bis(diethylamino)
Internal Xanthylium salt, 3,6-bis[[5-carboxy-2-[(methylsulphonyl)amino]phenyl]amino]-9-(2-carboxyphenyl)
Internal Xanthylium salt, 9-(2-carboxyphenyl)-3,6-bis[ethyl[2-[(ethylsulphonyl)amino]phenyl]amino]
Internal Xanthylium salt, 3-[ethyl[2-methyl-5-[(methylsulphonyl)amino]phenyl]amino]-6-[[2-methyl-5-[-(methylsulphonyl)amino]phenyl](3-sulphopropyl)amino]-9-(2-sulphophenyl), monosodium salt
Internal Xanthylium salt, 3,6-bis[methyl[2-[(methylsulphonyl)amino]phenyl]amino]-9-(2-sulphophenyl)
Internal Xanthylium salt, 3,6-bis[ethyl[2-methyl-5-[(methylsulphonyl)amino]phenyl]amino]-9-(2-sulphophenyl)
Internal Xanthylium salt, 3-[ethyl(2-methylphenyl)amino]-6-[[2-methyl-5-[(methylsulphonyl)amino]phenyl]amino]-9-(2-sulphophenyl)
Internal Xanthylium salt, 3-[(2,6-dimethylphenyl)amino]-9-(2,4-disulphophenyl)-6-[[2-[(methylsulphonyl)amino]phenyl]amino], monosodium salt
Internal Xanthylium salt, 3-amino-6-[[2-chloro-4-[2-[(methylsulphonyl)amino]phenoxy]phenyl]amino]-9-(2-sulphophenyl)
Internal Xanthylium salt, 3-amino-6-[[2-fluoro-4-[2-[(methylsulphonyl)amino]phenoxy]phenyl]amino]-9-(2-sulphophenyl)
Internal Xanthylium salt, 3-[(4-cyano-2-methoxyphenyl)amino]-6-[[4-[(methylsulphonyl)amino]-2-propoxyphenyl]amino]-9-(2-sulphophenyl)
Internal Xanthylium salt, 3-amino-6-[[2-methoxy-5-[2-[(methylsulphonyl)amino]phenoxy]phenyl]amino]-9-(2-sulphophenyl)
Internal Xanthylium salt, 3-[[5-(aminosulphonyl)-2-methoxyphenyl]amino]-6-[[4-[(ethoxycarbonyl)amino]-2-methoxyphenyl]amino]-9-(2-sulphophenyl)
Internal Xanthylium salt, 3,6-bis[ethyl[2-[(methylsulphonyl)-amino]ethyl]amino]-9-(2,4,5-tricarboxyphenyl)
Internal Xanthylium salt, 9-(2,4-disulphophenyl)-3,6-bis[ethyl[2-[(methylsulphonyl)amino]ethyl]amino] monolithium salt
Internal Xanthylium salt, 3,6-bis[ethyl[2-[(methylsulphonyl)amino]ethyl]amino]-9-(2-sulphophenyl)
Internal Xanthylium salt, 3-[[[[(5-carboxypentyl)amino]-sulphonyl]-2-methylphenyl]amino]-9-(2-carboxyphenyl)-6-[(2-methylphenyl)amino]
Internal Xanthylium salt, 9-(2-carboxyphenyl)-3-[[4-[[(3-carboxypropyl)amino]sulphonyl]-2,6-dimethylphenyl]amino]-6-[(2,6-dimethylphenyl)amino]
Internal Xanthylium salt, 3,6-bis[[4-[[(2-carboxyphenyl)-amino]sulphonyl]-2,6-dimethylphenyl]amino]-9-(2-sulphophenyl)
Internal Xanthylium salt, 3,6-bis[5-[(dodecylamino)sulphonyl]-2,3-dihydro-1H-indol-1-yl]-9-(2-sulphophenyl)
Internal Xanthylium salt, 3-[[4-[(dimethylamino)-sulphonyl]phenyl]methylamino]-6-(methylphenylamino)-9-(2-sulphophenyl)
Internal Xanthylium salt, 3,6-bis[[4-[(dimethylamino)-sulphonyl]phenyl]amino]-9-(2-sulphophenyl)
Internal Xanthylium salt, 3-chloro-6-[[4-[(dimethylamino)sulphonyl]phenyl]amino]-9-(2-sulphophenyl)
Internal Xanthylium salt, 3,6-bis[[4-[(dimethylamino)sulphonyl]phenyl]methylamino]-9-(2-sulphophenyl)
Internal Xanthylium salt, 3-[[4-[(dimethylamino)sulphonyl]phenyl]amino]-6-(phenylamino)-9-(2-sulphophenyl)
Internal Xanthylium salt, 3,6-bis[[4-[(dimethylamino)sulphonyl]-2-methoxyphenyl]amino]-9-(2-sulphophenyl)
Internal Xanthylium salt, 3,6-bis[[4-(aminosulphonyl)-phenyl]amino]-9-(2-sulphophenyl)
Internal salt of 9-{4-[bis(2-hydroxyethyl)sulphamoyl]-2-sulphophenyl}-3,6-bis(diethylamino)xanthenylium
Internal salt of 3,6-bis(diethylamino)-9-(4-dipropylsulphamoyl-2-sulphophenyl)xanthenylium
Internal salt of 3,6-bis(diethylamino)-9-[4-(4-methylpiperazine-1-sulphonyl)-2-sulphophenyl]-xanthenylium
Internal salt of 3,6-bis(diethylamino)-9-[4-(3-imidazol-1-ylpropylsulphamoyl)-2-sulphophenyl]xanthenylium
Internal salt of 9-{2-[bis(2-hydroxyethyl)sulphamoyl]-4-sulphophenyl}-3,6-bis(diethylamino)xanthenylium
Internal salt of 3,6-bis(diethylamino)-9-[2-(4-methylpiperazine-1-sulphonyl)-4-sulphophenyl]-xanthenylium.

4. Composition according to any one of the preceding claims, **characterised in that** the compound(s) according to formula (I) and/or the addition salt(s) represent 0.0001 to 10% by weight of the total weight of the dyeing composition, preferably 0.005 to 10% by weight and, more preferably, 0.01 to 6% by weight of the total weight of the dyeing composition.

5. Composition according to any one of the preceding claims, **characterised in that** it also contains one or more direct dye(s) different to the compounds according to formulas (I).

6. Composition according to claim 5, **characterised in that** the direct dye(s) different to the compounds according to formula (I) are selected from neutral, acidic or cationic nitro benzene direct dyes, neutral, acidic or cationic azo direct dyes, neutral, acidic or cationic quinone, particularly anthraquinone, direct dyes, azine direct dyes, triarylmethane direct dyes, indoamine direct dyes, natural direct dyes and xanthene direct dyes different to the compounds according to formula (I).

7. Composition according to any one of claims 5 and 6, **characterised in that** the direct dyes different to the compounds according to formulas (I) represents 0.001 to 20% by weight, preferably 0.005 to 10% by weight of the total weight of the dyeing composition.

8. Composition according to any one of the preceding claims, **characterised in that** it also contains one or more oxidation base(s) and optionally one or more coupling agent (s).

9. Composition according to claim 8, **characterised in that** the oxidation base(s) are selected from the paraphenylenediamines, bis-phenylalkylenediamines, para-aminophenols, ortho-aminophenols, heterocyclic bases and the addition salts thereof.

10. Composition according to claim 8,
**characterised in that** the coupling agent(s) are selected from metaphenylenediamine coupling agents, meta-aminophenol coupling agents, metadiphenol coupling agents, naphthalene-based coupling agents, heterocyclic coupling agents and the addition salts thereof.

11. Composition according to any one of claims 8 to 10, **characterised in that** the oxidation base(s) and/or the coupling agent(s) are each present in a quantity of 0.001 to 10% by weight, preferably 0.005 to 6% by weight of the total weight of the dyeing composition.

12. Composition according to any one of the preceding claims, **characterised in that** it has a pH of 3 to 12, preferably 5 to 11, more preferably 6 to 8.5.

13. Direct dyeing method of keratin fibres and particularly human keratin fibres, such as hair, comprising the application of at least one dyeing composition according to any of claims 1 to 12 containing at least one compound according to formula (I) on the keratin fibres followed by the observation of a leave-in time followed by the rinsing of the fibres.

14. Method according to claim 13, **characterised in that** the leave-in time is 3 to 50 minutes, preferably 5 to 30 minutes.

15. Dyeing method of keratin fibres and particularly human keratin fibres, such as hair, wherein, on said fibres, at least one dyeing composition according to any one of claims 1 to 12 comprising at least one compound according to formula (I) and optionally at least one oxidation base and optionally one or more coupling agent(s) are applied, the colour being developed at an acidic, neutral or alkaline pH, using an oxidizing agent.

16. Method according to claim 15, **characterised in that** the oxidizing agent is selected from hydrogen peroxide, urea peroxide, alkaline metal bromates, persalts such as perborates and persulphates, peracids and oxidase enzymes, such as peroxidases, 2-electron oxido-reductases, such as uricases and 4-electron oxygenases, such as laccases.

17. Device having several compartments comprising a first compartment containing a dyeing composition according to any one of claims 1 to 12, comprising at least one compound according to formula (I) and optionally at least one oxidation base and optionally one or more coupling agents, and a second compartment containing an oxidizing composition.

18. Device having several compartments comprising a first compartment which contains a dyeing composition according to any one of claims 1 to 12, comprising at least one compound according to formula (I), a second compartment contains a dyeing composition comprising at least one oxidation base and optionally at least one coupling agent, and a third compartment contains a oxidizing composition.

## Patentansprüche

1. Zusammensetzung zum Färben von Keratinfasern, die in einem zum Färben geeigneten, kosmetischen Medium mindestens einen Farbstoff enthält, der unter den Verbindungen, die der folgenden Formel (I) entsprechen, deren mesomeren Formen und deren Oligomeren ausgewählt ist: 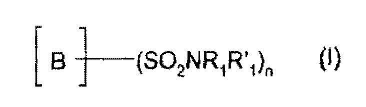 wobei B bedeutet: 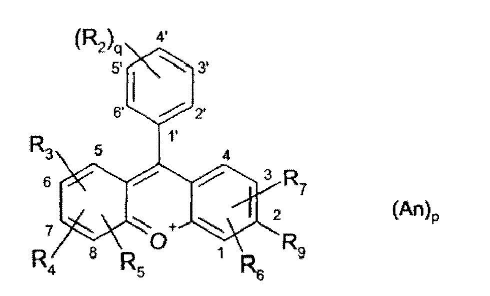 worin die Gruppen R₁ und R'₁, die gleich oder verschieden sind, bedeuten:
- ein Wasserstoffatom,
- eine Alkylgruppe,
- eine Thioalkylgruppe,
- eine Alkoxygruppe,
- eine Aminoalkylgruppe,
- eine Trialkylammonioalkylgruppe,
- eine Carboxyalkylgruppe,
- eine Carboxyaminoalkylgruppe,
- eine Aminoalkoxypolyalkoxygruppe, bei der die Alkoxygruppen der Polyalkoxygruppe, die gleich oder verschieden sind, jede 1 bis 4 Kohlenstoffatome enthalten,
- eine Alkoxycarbonylgruppe,
- eine Alkoxycarbonylalkylgruppe,
- eine Alkoxyalkylgruppe,
- eine Acylgruppe,
- eine Arylgruppe und vorzugsweise Phenyl,
- eine Arylalkylgruppe und vorzugsweise Benzyl,
- einen 5- bis 12-gliedrigen und vorzugsweise 5- bis 7-gliedrigen Heterocyclus mit einem Stickstoffatom, der gesättigt oder ungesättigt ist und gegebenenfalls mindestens ein weiteres Heteroatom enthält, das unter Sauerstoff, Stickstoff oder Schwefel ausgewählt ist, wobei der Heterocyclus gegebenenfalls substituiert ist,
wobei R'₁ auch die folgende Gruppe bedeuten kann: 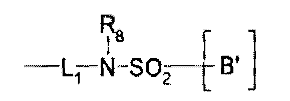 worin bedeuten:
- R₈ eine Gruppe, die unter den Gruppen ausgewählt ist, wie sie oben für R₁ und R'₁ definiert sind,
- L₁ eine zweiwertige Kohlenwasserstoffgruppe, die 1 bis 16 Kohlenstoffatome aufweist und gegebenenfalls durch mindestens ein Heteroatom, das unter Stickstoff, Sauerstoff oder Schwefel ausgewählt ist, und/oder durch eine Gruppe unterbrochen ist, die mindestens ein Heteroatom enthält, wie beispielsweise CO, SO₂,
- B' eine Gruppe der Formel (B), wobei B' mit B identisch ist oder nicht, die Gruppen R₁ und R'₁ können gegebenenfalls gemeinsam mit dem Stickstoffatom, an das sie gebunden sind, einen vorzugsweise gesättigten, 5- bis 12- gliedrigen und vorzugsweise 5- bis 7-gliedrigen Heterocyclus bilden, der gegebenenfalls mindestens ein weiteres Heteroatom enthält, das unter Sauerstoff, Stickstoff oder Schwefel ausgewählt ist, wobei der Heterocyclus gegebenenfalls substituiert ist;
- R₂ bedeutet eine Gruppe, die unter dem Wasserstoffatom, Halogenatomen, wie F, Cl, Br und I, Alkoxygruppen, Alkylgruppen, der Sulfonsäuregruppe, der Carboxygruppe ausgewählt ist;
- die Gruppen R₃, R₄, R₅, R₆, R₇ und R₉ bedeuten jeweils unabhängig voneinander eine Gruppe, die ausgewählt ist unter dem Wasserstoffatom; Halogenatomen, wie F, Cl, Br und I; Alkoxygruppen; Alkylgruppen; Aminogruppen; Aminogruppen, die mit einer Gruppe mono- oder disubstituiert sind, die unter der Benzylgruppe, Arylgruppen und Alkylgruppen ausgewählt sind, wie den Gruppen Monoarylamino, Diarylamino, Monoalkylamino, (Dialkyl)amino, wobei die Substituenten der Aminogruppe, wie die Alkylgruppen, gemeinsam mit dem Stickstoffatom, an das sie gebunden sind, einen vorzugsweise gesättigten, 5- bis 12- gliedrigen und vorzugsweise 5- bis 7-gliedrigen Heterocyclus bilden können, der gegebenenfalls mindestens ein weiteres Heteroatom enthält, das unter Stickstoff, Sauerstoff und Schwefel ausgewählt ist, wobei der Heterocyclus gegebenenfalls substituiert ist; oder wobei einer oder die beiden Substituenten der Aminogruppe, wie die Alkylgruppen, gemeinsam mit einem Substituenten, der sich zu der mono- oder disubstituierten Aminogruppe, wie Monoalkylamino oder (Dialkyl)amino, in ortho-Stellung befindet, einen 5- bis 6-gliedrigen Heterocyclus bilden können, der gegebenenfalls ferner mindestens ein weiteres Heteroatom enthält, das unter Stickstoff, Sauerstoff und Schwefel ausgewählt ist,
wobei der Heterocyclus gegebenenfalls substituiert ist;
wobei die Gruppe(n) -SO₂NR₁R'₁ und/oder -LiNR₈SO₂- entweder direkt an einen oder mehrere der Ringe gebunden sind, die die Substituenten R₂ bis R₉ tragen, oder über einen oder mehrere der Substituenten R₂ bis R₉,
der Koeffizient n bedeutet ein ganze Zahl im Bereich von 1 bis 4 und vorzugsweise 1;
der Koeffizient q bedeutet ein ganze Zahl im Bereich von 1 bis 3 und vorzugsweise 1;
An bedeutet ein ein- oder mehrwertiges Anion oder ein Gemisch von Anionen;
der Koeffizient p ist 0 oder 1;
An und p sind so gewählt, dass die Verbindung elektrisch neutral ist.

2. Zusammensetzung zum Färben nach Anspruch 1, wobei in der Formel (I) die Gruppen R₁ und R'₁, die gleich oder verschieden sind, bedeuten:
- ein Wasserstoffatom,
- eine geradkettige oder verzweigte C₁₋₁₆-Alkylgruppe oder cyclische C₃₋₁₆-Alkylgruppe, die gegebenenfalls mit einer oder mehreren Gruppen substituiert sind, die gleich oder verschieden sind und die unter den Gruppen Amino, Sulfonsäure und Alkoxy ausgewählt sind,
- eine geradkettige oder verzweigte C₁₋₁₆-Hydroxyalkylgruppe oder cyclische C₃₋₁₆-Hydroxyalkylgruppe,
- eine geradkettige oder verzweigte C₁₋₁₆-Thioalkylgruppe oder cyclische C₃₋₁₆-Thioalkylgruppe,
- eine geradkettige oder verzweigte C₁₋₁₆-Alkoxygruppe,
- eine geradkettige oder verzweigte C₁₋₁₆-Aminoalkylgruppe oder cyclische C₃₋₁₆-Aminoalkylgruppe,
- eine Trialkylammonioalkylgruppe, bei der die Alkylgruppen, die gleich oder verschieden sind, 1 bis 16 Kohlenstoffatome aufweisen,
- eine geradkettige oder verzweigte Halogenalkylgruppe, bei der die Alkylgruppe, die geradkettig oder verzweigt ist, 1 bis 16 Kohlenstoffatome aufweist,
- eine geradkettige oder verzweigte Carboxy C₁₋₁₆ alkylgruppe oder cyclische Carboxy C₃₋₁₆ alkylgruppe,
- eine geradkettige oder verzweigte Carboxy amino C₁₋₁₆alkylgruppe oder cyclische Carboxyamino C₃₋₁₆ alkylgruppe,
- eine Aminoalkoxypolyalkoxygruppe, bei der die Alkoxygruppen, die gleich oder verschieden und linear oder verzweigt sind, 1 bis 4 Kohlenstoffatome aufweisen,
- eine Alkoxycarbonylgruppe, bei der die Alkoxygruppe, die linear oder verzweigt ist, 1 bis 16 Kohlenstoffatome aufweist,
- eine Alkoxycarbonylalkylgruppe, bei der die geradkettige oder verzweigte Alkoxygruppe 1 bis 16 Kohlenstoffatome aufweist und die geradkettige oder verzweigte Alkylgruppe 1 bis 16 Kohlenstoffatome aufweist,
- eine Alkoxyalkylgruppe, bei der die geradkettige oder verzweigte Alkoxygruppe 1 bis 16 Kohlenstoffatome aufweist und die geradkettige oder verzweigte Alkylgruppe 1 bis 16 Kohlenstoffatome aufweist,
- eine Acylgruppe, bei der die geradkettige oder verzweigte Alkylgruppe 1 bis 16 Kohlenstoffatome aufweist,
- eine Arylgruppe und vorzugsweise Phenyl, die gegebenenfalls mit einer oder mehreren Gruppen substituiert ist, die unter den Gruppen Amino, Sulfo und Alkoxy ausgewählt sind,
- eine Arylalkylgruppe und vorzugsweise Benzyl, die gegebenenfalls mit einer oder mehreren Gruppen substituiert ist, die unter den Gruppen Amino, Sulfo und Alkoxy ausgewählt sind,
- einen gesättigten oder ungesättigten, 5- bis 12- gliedrigen und vorzugsweise 5- bis 7-gliedrigen Heterocyclus mit einem Stickstoffatom, der gegebenenfalls mindestens ein weiteres Heteroatom enthält, das unter Sauerstoff, Stickstoff oder Schwefel ausgewählt ist, wobei der Heterocyclus gegebenenfalls mit einer oder mehreren Gruppe(n) substituiert ist, die gleich oder verschieden sind und unter den geradkettigen oder verzweigten C₁₋₁₆-Alkylgruppen oder C₁₋₁₆-Hyroxyalkylgruppen, Carboxygruppen, geradkettigen oder verzweigten Alkoxycarbonylgruppen, bei denen die Alkoxygruppe 1 bis 16 Kohlenstoffatome aufweist, Aminoalkylcarbamoylgruppen mit einer geradkettigen oder verzweigten C₁₋₁₆-Alkylgruppe ausgewählt sind;
R₂ bedeutet eine Gruppe, die unter dem Wasserstoffatom, Halogenatomen, wie F, Cl, Br und I, Alkoxygruppen, Alkylgruppen, der Sulfonsäuregruppe, der Carboxygruppe ausgewählt ist,
die Gruppen R₃, R₄, R₅, R₆, R₇ und R₉ , die gleich oder verschieden sind, bedeuten jeweils unabhängig voneinander eine Gruppe, die ausgewählt ist unter dem Wasserstoffatom; Halogenatomen, wie F, Cl, Br und I; Alkoxygruppen; Alkylgruppen; Aminogruppen; Aminogruppen, die mit einer oder mehreren Gruppe(n) mono- oder disubstituiert sind, die gleich oder verschieden sind und die unter der Benzylgruppe, Arylgruppen und Alkylgruppen ausgewählt ist (sind), wie den Gruppen Monoarylamino, Diarylamino, Monoalkylamino, (Dialkyl)amino, wobei die Substituenten der Aminogruppe, wie die Alkylgruppen, gemeinsam mit dem Stickstoffatom, an das sie gebunden sind, einen vorzugsweise gesättigten, 5- bis 12-gliedrigen und vorzugsweise 5- bis 7-gliedrigen Heterocyclus bilden können, der gegebenenfalls mindestens ein weiteres Heteroatom enthält, das unter Stickstoff, Sauerstoff und Schwefel ausgewählt ist, wobei der Heterocyclus gegebenenfalls substituiert sein kann; oder wobei einer oder die beiden Substituenten der Aminogruppe, wie die Alkylgruppen, gemeinsam mit einem Substituenten, der sich zu der mono- oder disubstituierten Aminogruppe, wie Monoalkylamino oder (Dialkyl)amino, in ortho-Stellung befindet, einen 5- bis 6-gliedrigen Heterocyclus bilden können, der gegebenenfalls ein oder mehrere weitere Heteroatome enthalten kann, die unter Stickstoff, Sauerstoff und
Schwefel ausgewählt sind, wobei der Heterocyclus gegebenenfalls substituiert sein kann;
geradkettigen oder verzweigten C₁₋₁₆-Sulfoalkylgruppen und vorzugsweise C₁₋₁₀-Sulfoalkylgruppen;
geradkettigen oder verzweigten C₁₋₁₆-Hydroxyalkylgruppen und vorzugsweise C₁₋₁₀-Hyroxyalkylgruppen;
Mono- und Diarylaminogruppen, bei denen der Arylteil gegebenenfalls mit einem oder mehreren Substituenten substituiert ist, die gleich oder verschieden sind und die ausgewählt sind unter Halogenatomen; geradkettigen oder verzweigten C₁₋₁₆-Alkylgruppen und vorzugsweise C₁₋₁₀-Alkylgruppen; geradkettigen oder verzweigten C₁₋₁₆-Alkoxygruppen und vorzugsweise C₁₋₁₀-Alkoxygruppen; Aryloxygruppen, die gegebenenfalls substituiert sind; Mesyl (CH₃-SO₂-) ; Amino; Aminogruppen, die mit einer oder mehreren Gruppen mono- oder disubstituiert sind, die gleich oder verschieden sind und die unter den geradkettigen oder verzweigten C₁₋₄-Alkylgruppen, Phenyl ausgewählt sind, wobei der oder die Substituenten der Aminogruppe, wie die Alkylgruppen, gemeinsam mit dem Stickstoffatom der Aminogruppe, an das sie gebunden sind, einen vorzugsweise gesättigten, 5- bis 12-gliedrigen und vorzugsweise 5- bis 7-gliedrigen Heterocyclus bilden können, der gegebenenfalls ein weiteres Heteroatom enthält, das unter Sauerstoff, Stickstoff oder Schwefel ausgewählt ist; wobei der Heterocyclus gegebenenfalls substituiert ist, wobei der Heterocyclus gegebenenfalls mit einem aromatischen, vorzugsweise 6-gliedrigen Ring kondensiert sein kann, wobei dieser gegebenenfalls substituiert ist;
L₁ bedeutet eine geradkettige oder verzweigte C₁₋₁₆-Alkylgruppe, die gegebenenfalls durch mindestens ein Heteroatom, das unter Stickstoff, Sauerstoff und Schwefel ausgewählt ist, und/oder durch mindestens eine Gruppe unterbrochen ist, die mindestens ein Heteroatom enthält, das unter Stickstoff, Sauerstoff und Schwefel ausgewählt ist;
An bedeutet ein organisches oder anorganisches Anion oder ein Gemisch von organischen oder anorganischen Anionen, die beispielsweise unter einem Halogenid, wie Chlorid, Bromid, Fluorid, Iodid; Hydroxid; Sulfat; Hydrogensulfat; einem Alkylsulfat, bei dem der Alkylteil, der geradkettig oder verzweigt ist, 1 bis 6 Kohlenstoffatome aufweist, wie Methylsulfat und Ethylsulfat; Carbonaten und Hydrogencarbonaten; Carbonsäuresalzen, wie Formiat, Acetat, Citrat, Tartrat, Oxalat; Alkylsulfonaten, bei denen der Alkylteil, der geradkettig oder verzweigt ist, 1 bis 6 Kohlenstoffatome aufweist, wie Methylsulfonat; Arylsulfonaten, bei denen der Arylteil, vorzugsweise Phenyl, gegebenenfalls mit einer oder mehreren C₁₋₄-Alkylgruppen substituiert ist, wie z.B. 4-Toluylsulfonat; Alkylsulfonylgruppen, wie Mesylat, ausgewählt sind.

3. Zusammensetzung zum Färben nach Anspruch 1 oder 2, **dadurch gekennzeichnet, dass** sie eine Verbindung der Formel (I) enthält, die unter den folgenden Verbindungen ausgewählt ist:
inneres Salz von 9-[4-[[(3-Amino-4-sulfophenyl)amino]sulfonyl]-2-sulfophenyl]-3,6-bis(dimethylamino)-xanthylium,
inneres Salz von 3,6-Bis(diethylamino)-9-[2-sulfo-4-[[(4-sulfophenyl)amino]sulfonyl]phenyl]-xanthylium,
inneres Salz von 3,6-Bis(diethylamino)-9-[4-[[[2-(ethylthio)-ethyl]amino]sulfonyl]-2-sulfophenyl]-xanthylium,
inneres Salz von 3,6-Bis(diethylamino)-9-[4-[[(4-methoxy-2-sulfophenyl)amino]sulfonyl]-2-sulfophenyl]-xanthylium,
inneres Salz von 3,6-Bis(diethylamino)-9-[4-[[(2-methoxy-4-sulfophenyl)amino]sulfonyl]-2-sulfophenyl]-xanthylium,
inneres Salz von 9-[4-[[(3-Amino-2-sulfophenyl)amino]sulfonyl]-2-sulfophenyl]- 3,6-bis(diethylamino)-xanthylium,
inneres Salz von 9-[4-[[(3-Amino-4-sulfophenyl)amino]sulfonyl]-2-sulfophenyl]- 3,6-bis(diethylamino)-xanthylium,
9-[2-[(4-Carboxy-1-piperidinyl)sulfonyl]phenyl]-3,6-bis(phenylamino)-xanthyliumchlorid,
3,6-Bis(2,3-dihydro-1H-indol-1-yl)-9-[2-[[4-(ethoxycarbonyl)-1-piperidinyl] sulfonyl]phenyl]-xanthyliumchlorid,
inneres Salz von 9,9'-[Dithiobis[2,1-ethandiyliminosulfonyl-(2-sulfo-4,1-phenylen)]]bis[3,6-bis(diethylamino)-xanthylium,
9-[2-[(Dihexylamino)sulfonyl]phenyl]-3,6-bis(2,3-dihydro-2,3,3-trimethyl-1H-indol-1-yl)-xanthyliumchlorid,
3,6-Bis[(2-chlorphenyl)methylamino]-9-[2-[(dihexylamino)-sulfonyl]phenyl]-xanthyliumchlorid,
inneres Salz von 9-[4-[[[2-(Carboxyamino)ethyl]amino]sulfonyl]-2-sulfophenyl]-3,6-bis(diethylamino)-xanthylium,
inneres Salz von 9-[2-[[[2-(Carboxyamino)ethyl]amino]sulfonyl]-4-sulfophenyl]-3,6-bis(diethylamino)-xanthylium,
inneres Salz von 9-[4-[[(6-Aminohexyl)amino]sulfonyl]-2-sulfophenyl]-3,6-bis(diethylamino)-xanthylium,
9-[2-[[4-[[(5-Aminopentyl)amino]carbonyl]-1-piperidinyl]-sulfonyl]phenyl]-3,6-bis(methylphenylamino)-xanthyliumchlorid,
inneres Salz von 9-[2-[(4-Carboxy-1-piperidinyl)sulfonyl]-phenyl]-3,6-bis[methyl(4-sulfophenyl)amino]-xanthylium,
9-[2-[(4-Carboxy-l-piperidinyl)sulfonyl]phenyl]-3,6-bis(methylphenylamino)-xanthyliumchlorid,
9-[2-[[3-(Hydroxymethyl)-1-piperidinyl]sulfonyl]phenyl]-3,6-bis(methylphenylamino)-xanthyliumchlorid,
9-[2-[(4-carboxy-1-piperidinyl)sulfonyl]phenyl]-3,6-bis(methylphenylamino)-xanthylium,
inneres Salz von 9-[4-[[[3-[2-[2-(3-aminopropoxy)ethoxy]-ethoxy]propyl]amino]sulfonyl]-2-sulfophenyl]-3,6-bis(diethylamino)-xanthylium,
inneres Salz von 9-[4-[[(2-Aminoethyl)amino]sulfonyl]-2-sulfophenyl]-3,6-bis(diethylamino)-xanthylium,
inneres Salz von 9-[2-[[(5-Carboxypentyl)amino]sulfonyl]-4-sulfophenyl]-3,6-bis(diethylamino)-xanthylium,
inneres Salz von 3,6-Bis(diethylamino)-9-[2-[[(6-methoxy-6-oxohexyl)amino]sulfonyl]-4-sulfophenyl-xanthylium,
inneres Salz von 3,6-Bis(diethylamino)-9-[4-[[(6-methoxy-6-oxohexyl)amino]sulfonyl]-2-sulfophenyl]-xanthylium,
inneres Salz von 3,6-Bis(diethylamino)-9-[4-[(methylamino)-sulfonyl]-2-sulfophenyl]-xanthylium-Dihydrat,
3,6-Bis(diethylamino)-9-[2-[(methylamino)sulfonyl]-4-sulfophenyl]-xanthyliumchlorid,
9-[2-(Aminosulfonyl)phenyl]-3-[(2-sulfophenyl)amino]-xanthyliumchlorid, Mononatriumsalz,
inneres Salz von 3,6-Bis(diethylamino)-9-[4-(1-piperazinylsulfonyl)-2-sulfophenyl]-xanthylium,
3,6-Bis[[2-(1,1-dimethylethyl)phenyl]amino]-9-[2-[(ethylamino)-sulfonyl]phenyl]-xanthyliumbromid,
9-[2-[(Acetylmethylamino)sulfonyl]phenyl]-3,6-bis(methylphenylamino)-xanthyliumhexafluorphosphat,
9-[2-[(Acetylmethylamino)sulfonyl]phenyl]-3,6-bis(methylphenylamino)-xanthylium,
3-[(2-Brom-5-sulfophenyl)amino]-9-[2-[[(2-hydroxyethyl)amino]-sulfonyl]phenyl]-6-[(1,1,3,3-tetramethylbutyl)amino]-xanthyliumchlorid, Mononatriumsalz,
3-[(2-Fluor-5-sulfophenyl)amino]-9-[2-[[(2-hydroxyethyl)-amino]sulfonyl]phenyl]-6-[(1,1,3,3-tetramethylbutyl)amino]-xanthyliumchlorid, Mononatriumsalz,
3-[(2,6-Dimethylphenyl)amino]-9-[2-[[(2-hydroxyethyl)-amino]sulfonyl]phenyl]-6-[(2-methoxy-5-sulfophenyl)amino]-xanthyliumchlorid, Mononatriumsalz,
inneres Salz von 9-[4-[[(5-Carboxypentyl)amino]sulfonyl]-2-sulfophenyl]-3,6-bis(diethylamino)-xanthylium,
inneres Salz von 9-[4-[(Cyclohexylamino)sulfonyl]-2-sulfophenyl]-3,6-bis(diethylamino)-xanthylium,
inneres Salz von 3,6-Bis(diethylamino)-9-[4-[[(3-ethoxypropyl)-amino]sulfonyl]-2-sulfophenyl]-xanthylium,
inneres Salz von 3,6-Bis(diethylamino)-9-[4-[[[3-[(2-ethylhexyl)-oxy]propyl]amino]sulfonyl]-2-sulfophenyl]-xanthylium,
inneres Salz von 9-[4-[[(3-Carboxypropyl)amino]sulfonyl]-2-sulfophenyl]-3,6-bis(diethylamino)-xanthylium,
9-[2-[(Acetylmethylamino)sulfonyl]phenyl]-3,6-bis[2,3-dihydro-5-[[[3-(trimethylammonio)propyl]amino]sulfonyl]-1H-indol-1-yl]-xanthylium,
3,3'-[[9-[2-[(Acetylmethylamino)sulfonyl]phenyl]-9H-xanthen-3,6-diyl]bis[(2,3-dihydro-1H-indol-1,5-diyl)sulfonylimino]]-bis[N,N,N-trimethyl]-1-propanaminiumdiiodid,
inneres Salz von 9-[4-[[[2-[[(2-Aminoethoxy)methoxy]methoxy]-ethyl]amino]sulfonyl]-2-sulfophenyl]-3,6-bis(diethylamino)-xanthylium,
inneres Salz von 3,6-Bis(diethylamino)-9-[2-[(methylamino)sulfonyl]-4-sulfophenyl]-xanthylium,
inneres Salz von 3,6-Bis(diethylamino)-9-[4-[(methylamino)-sulfonyl]-2-sulfophenyl]-xanthylium,
inneres Salz von 9-[4-[[(11-Carboxyundecyl)amino]sulfonyl]-2-sulfophenyl]-3,6-bis(diethylamino)-xanthylium,
9-[2-(Aminosulfonyl)phenyl]-3,6-bis(diethylamino)-xanthylium-4-methylbenzolsulfonat,
inneres Salz von [9-[4-[Bis(2-hydroxyethyl)sulfamoyl]-2-sulfophenyl]-6-(diethylamino)-3H-xanthen-3-yliden]diethylammoniumhydroxid,
inneres Salz von [9-[4-[(2-Chlorethyl)sulfamoyl]-2-sulfophenyl]-6-(diethylamino)-3H-xanthen-3-yliden]diethylammoniumhydroxid,
inneres Salz von [9-[4-[Bis(2-chlorethyl)sulfamoyl]-2-sulfophenyl]-6-(diethylamino)-3H-xanthen-3-yliden]diethylammoniumhydroxid,
inneres Salz von [6-(Diethylamino)-9-[4-(phenylsulfamoyl)-2-sulfophenyl]-3H-xanthen-3-yliden]diethylammoniumhydroxid,
inneres Salz von 3,6-Bis(diethylamino)-9-[4-[[(2-hydroxyethyl)-amino]sulfonyl]-2-sulfophenyl]-xanthylium,
inneres Salz von [6-(Diethylamino)-9-(4-sulfamoyl-2-sulfophenyl)-3H-xanthen-3-yliden]diethylammoniumhydroxid,
inneres Salz von 3,6-Bis[5-[(dodecylamino)sulfonyl]-2,3-dihydro-1H-indol-1-yl]-9-(2-sulfophenyl)-xanthylium,
3,6-Bis(2,3-dihydro-1H-indol-1-yl)-9-[2-[(methylamino)-sulfonyl]phenyl]-xanthylium,
9-[2-[(Methylamino)sulfonyl]phenyl]-3-[methyl[2-(methylsulfonyl)phenyl]amino]-6-[methyl[4-(methylsulfonyl)phenyl]-amino]-xanthylium,
inneres Salz von 3-[[4-[(Dimethylamino)sulfonyl]phenyl]-methylamino]-6-(methylphenylamino)-9-(2-sulfophenyl)-xanthylium,
9-[2-(Aminosulfonyl)phenyl]-3,6-bis(diethylamino)-xanthyliumchlorid,
inneres Salz von 3,6-Bis[[5-carboxy-2-[(methylsulfonyl)-amino]phenyl]amino]-9-(2-carboxyphenyl)-xanthylium,
inneres Salz von 9-(2-Carboxyphenyl)-3,6-bis[ethyl[2-[(ethylsulfonyl)amino]phenyl]amino]-xanthylium,
inneres Salz von 3-[ethyl[2-methyl-5-[(methylsulfonyl)-amino]phenyl]amino]-6-[[2-methyl-5-[(methylsulfonyl)amino]-phenyl](3-sulfopropyl)amino]-9-(2-sulfophenyl)-xanthylium, Mononatriumsalz,
inneres Salz von 3,6-Bis[methyl[2-[(methylsulfonyl)-amino]phenyl]amino]-9-(2-sulfophenyl)-xanthylium,
inneres Salz von 3,6-Bis[ethyl[2-methyl-5-[(methylsulfonyl)amino]phenyl]amino]- 9-(2-sulfophenyl)-xanthylium,
inneres Salz von 3-[Ethyl(2-methylphenyl)amino]-6-[[2-methyl-5-[(methylsulfonyl)amino]phenyl]amino]-9-(2-sulfophenyl)-xanthylium,
inneres Salz von 3-[(2,6-Dimethylphenyl)amino]-9-(2,4-disulfophenyl)-6-[[2-[(methylsulfonyl)amino]phenyl]amino]-xanthylium, Mononatriumsalz,
inneres Salz von 3-Amino-6-[[2-chlor-4-[2-[(methylsulfonyl)amino]phenoxy]phenyl]amino]-9-(2-sulfophenyl)-xanthylium,
inneres Salz von 3-Amino-6-[[2-fluor-4-[2-[(methylsulfonyl)amino]phenoxy]phenyl]amino]-9-(2-sulfophenyl)-xanthylium,
inneres Salz von 3-[(4-Cyano-2-methoxyphenyl)amino]-6-[[4-[(methylsulfonyl)amino]-2-propoxyphenyl]amino]-9-(2-sulfophenyl)-xanthylium,
inneres Salz von 3-Amino-6-[[2-methoxy-5-[2-[(methylsulfonyl)amino]phenoxy]phenyl]amino]-9-(2-sulfophenyl)-xanthylium,
inneres Salz von 3-[[5-(Aminosulfonyl)-2-methoxyphenyl]-amino]-6-[[4-[(ethoxycarbonyl)amino]-2-methoxyphenyl]amino]-9-(2-sulfophenyl)-xanthylium,
inneres Salz von 3,6-Bis[ethyl[2-[(methylsulfonyl)amino]ethyl]-amino]-9-(2,4,5-tricarboxyphenyl)-xanthylium,
inneres Salz von 9-(2,4-Disulfophenyl)-3,6-bis[ethyl[2-[(methylsulfonyl)amino]ethyl]amino]-xanthylium, Monolithiumsalz,
inneres Salz von 3,6-Bis[ethyl[2-[(methylsulfonyl)amino]ethyl]-amino]-9-(2-sulfophenyl)-xanthylium,
inneres Salz von 3-[[[[(5-Carboxypentyl)amino]sulfonyl]-2-methylphenyl]amino]-9-(2-carboxyphenyl)-6-[(2-methylphenyl)amino]-xanthylium,
inneres Salz von 9-(2-Carboxyphenyl)-3-[[4-[[(3-carboxypropyl)amino]sulfonyl]- 2,6-dimethylphenyl]amino]-6-[(2,6-dimethylphenyl)amino]-xanthylium,
inneres Salz von 3,6-Bis[[4-[[(2-carboxyphenyl)amino]sulfonyl]-2,6-dimethylphenyl]amino]-9-(2-sulfophenyl)-xanthylium,
inneres Salz von 3,6-Bis[5-[(dodecylamino)sulfonyl]-2,3-dihydro-1H-indol-1-yl]-9-(2-sulfophenyl)-xanthylium,
inneres Salz von 3-[[4-[(Dimethylamino)sulfonyl]phenyl]-methylamino]-6-(methylphenylamino)-9-(2-sulfophenyl)-xanthylium,
inneres Salz von 3,6-Bis[[4-[(dimethylamino)sulfonyl]-phenyl]amino]-9-(2-sulfophenyl)-xanthylium,
inneres Salz von 3-Chlor-6-[[4-[(dimethylamino)sulfonyl]-phenyl]amino]-9-(2-sulfophenyl)-xanthylium,
inneres Salz von 3,6-Bis[[4-[(dimethylamino)sulfonyl]phenyl]-methylamino]-9-(2-sulfophenyl)-xanthylium,
inneres Salz von 3-[[4-[(Dimethylamino)sulfonyl]phenyl]amino]-6-(phenylamino)-9-(2-sulfophenyl)-xanthylium,
inneres Salz von 3,6-Bis[[4-[(dimethylamino)sulfonyl]-2-methoxyphenyl]amino]-9-(2-sulfophenyl)-xanthylium,
inneres Salz von 3,6-Bis[[4-(aminosulfonyl)phenyl]amino]-9-(2-sulfophenyl)-xanthylium,
inneres Salz von 9-{4-[Bis-(2-hydroxy-ethyl)-sulfamoyl]-2-sulfophenyl}-3,6-bis-diethylamino-xanthenylium,
inneres Salz von 3,6-Bis-diethylamino-9-(4-dipropylsulfamoyl-2-sulfo-phenyl)-xanthenylium,
inneres Salz von 3,6-Bis-diethylamino-9-[4-(4-methylpiperazin-1-sulfonyl)-2-sulfo-phenyl]-xanthenylium,
inneres Salz von 3,6-Bis-diethylamino-9-[4-(3-imidazol-1-yl-propylsulfamoyl)-2-sulfo-phenyl]-xanthenylium,
inneres Salz von 9-{2-[Bis-(2-hydroxy-ethyl)-sulfamoyl]-4-sulfophenyl}-3,6-bis-diethylamino-xanthenylium,
inneres Salz von 3,6-Bis-diethylamino-9-[2-(4-methylpiperazin-1-sulfonyl)-4-sulfo-phenyl]-xanthenylium.

4. Zusammensetzung nach einem der vorhergehenden Ansprüche, **dadurch gekennzeichnet, dass** die Verbindung(en) der Formel (I) und/oder das oder die Additionssalze 0,0001 bis 10 Gew.-% des Gesamtgewichts der Farbmittelzusammensetzung, vorzugsweise 0,005 bis 10 Gew.-% und noch bevorzugter 0,01 bis 6 Gew.-% des Gesamtgewichts der Farbmittelzusammensetzung ausmachen.

5. Zusammensetzung nach einem der vorhergehenden Ansprüche, **dadurch gekennzeichnet, dass** sie ferner einen oder mehrere Direktfarbstoff(e) enthält, die von den Verbindungen der Formel (I) verschieden sind.

6. Zusammensetzung nach Anspruch 5, **dadurch gekennzeichnet, dass** der oder die Direktfarbstoff(e), die von den Verbindungen der Formel (I) verschieden sind, unter den neutralen, sauren oder kationischen nitrierten Direktfarbstoffen auf Benzolbasis, neutralen, sauren oder kationischen direktziehenden Azofarbstoffen, neutralen, sauren oder kationischen direktziehenden Chinon-Farbstoffen und insbesondere Anthrachinon-Farbstoffen, direktziehenden Azin-Farbstoffen, direktziehenden Triarylmethan-Farbstoffen, direktziehenden Indoaminfarbstoffen, direktziehenden natürlichen Farbstoffen und direktziehenden Xanthen-Farbstoffen ausgewählt sind, die von den Verbindungen der Formel (I) verschieden sind.

7. Zusammensetzung nach einem der Ansprüche 5 und 6, **dadurch gekennzeichnet, dass** der oder die Direktfarbstoff(e), die von den Verbindungen der Formel (I) verschieden sind, 0,001 bis 20 Gew.-% und vorzugsweise 0,005 bis 10 Gew.-% des Gesamtgewichts der Farbmittelzusammensetzung ausmachen.

8. Zusammensetzung nach einem der vorhergehenden Ansprüche, **dadurch gekennzeichnet, dass** sie ferner eine oder mehrere Oxidationsbase(n) und gegebenenfalls einen oder mehrere Kuppler enthält.

9. Zusammensetzung nach Anspruch 8, **dadurch gekennzeichnet, dass** die Oxidationsbase(n) unter den p-Phenylendiaminen, Bisphenylalkylendiaminen, p-Aminophenolen, o-Aminophenolen, heterocyclischen Basen und deren Additionssalzen ausgewählt sind.

10. Zusammensetzung nach Anspruch 8, **dadurch gekennzeichnet, dass** der oder die Kuppler unter den m-Phenylendiaminen, m-Aminophenolen, m-Dihydroxybenzolen, Naphthalinkupplern und heterocyclischen Kupplern und deren Additionssalzen ausgewählt sind.

11. Zusammensetzung nach einem der Ansprüche 8 bis 10, **dadurch gekennzeichnet, dass** die Oxidationsbase(n) und/oder der oder die Kuppler jede(r) in einer Menge von 0,001 bis 10 Gew.-% und vorzugsweise 0,005 bis 6 Gew.-% des Gesamtgewichts der Farbmittelzusammensetzung enthalten sind.

12. Zusammensetzung nach einem der vorhergehenden Ansprüche, **dadurch gekennzeichnet, dass** sie einen pH-Wert im Bereich von 3 bis 12, vorzugsweise 5 bis 11 und noch bevorzugter 6 bis 8,5 aufweist.

13. Verfahren für die Direktfärbung von Keratinfasern und insbesondere menschlichen Keratinfasern, wie Haaren, das umfasst, mindestens eine Farbmittelzusammensetzung nach einem der Ansprüche 1 bis 12, die mindestens eine Verbindung der Formel (I) enthält, auf die Keratinfasern aufzutragen und anschließend eine Einwirkzeit abzuwarten und dann die Fasern zu spülen.

14. Verfahren nach Anspruch 13, **dadurch gekennzeichnet, dass** die Einwirkzeit 3 bis 50 min und vorzugsweise 5 bis 30 min beträgt.

15. Verfahren zum Färben von Keratinfasern und insbesondere menschlichen Keratinfasern, wie Haaren, das umfasst, mindestens eine Farbmittelzusammensetzung nach einem der Ansprüche 1 bis 12, die mindestens eine Verbindung der Formel (I) und gegebenenfalls mindestens eine Oxidationsbase und gegebenenfalls einen oder mehrere Kuppler enthält, auf die Fasern aufzutragen und die Farbe bei einem sauren, neutralen oder alkalischen pH-Wert mit Hilfe eines Oxidationsmittels zu bilden.

16. Verfahren nach Anspruch 15, **dadurch gekennzeichnet, dass** das Oxidationsmittel unter Wasserstoffperoxid, Harnstoffperoxid, Alkalimetallbromaten, Salzen von Persäuren, wie Perboraten und Persulfaten, Persäuren und Oxidasen, wie Peroxidasen, Oxidoreductasen (2 Elektronen), wie Uricasen, und Oxygenasen (4 Elektronen), wie Laccasen, ausgewählt ist.

17. Vorrichtung mit mehreren Abteilungen, die eine erste Abteilung, die eine Farbmittelzusammensetzung nach einem der Ansprüche 1 bis 12 enthält, die mindestens eine Verbindung der Formel (I) und gegebenenfalls mindestens eine Oxidationsbase und gegebenenfalls einen oder mehrere Kuppler enthält, und eine zweite Abteilung umfasst, die eine oxidierende Zusammensetzung enthält.

18. Vorrichtung mit mehreren Abteilungen, die eine erste Abteilung, die eine Farbmittelzusammensetzung nach einem der Ansprüche 1 bis 12 enthält, die mindestens eine Verbindung der Formel (I) enthält, eine zweite Abteilung, die eine Farbmittelzusammensetzung enthält, die mindestens eine Oxidationsbase und gegebenenfalls einen oder mehrere Kuppler enthält, und eine dritte Abteilung umfasst, die eine oxidierende Zusammensetzung enthält.
